# EUROPEAN PATENT SPECIFICATION

(11) **EP 4 120 910 B1**
(45) Date of publication and mention of the grant of the patent: **04.11.2026**
(21) Application number: 21716026.6
(22) Date of filing: 11.03.2021
(51) Int. Cl.: A61B 5/11, G06F 3/16, H04R 1/10, H04R 5/00, H04R 25/00

(54) **POSTURE DETECTION USING HEARING INSTRUMENTS**
HALTUNGSERKENNUNG MIT HÖRGERÄTEN
DÉTECTION DE POSTURE À L'AIDE D'INSTRUMENTS AUDITIFS

(30) Priority: 16.03.2020 US 202062990182 P
(43) Date of publication of application: 25.01.2023
(73) Proprietor: Starkey Laboratories, Inc., Eden Prairie, MN 55344 (US)
(72) Inventor: BURWINKEL, Justin, Eden Prairie, Minnesota 55344 (US); ROZENMAN, Roy, Eden Prairie, Minnesota 55344 (US)
(74) Representative: Crowell & Moring U.K. LLP
(86) International application number: PCT/US2021/021940
(87) International publication number: WO 2021/188360

(56) References cited:
- US-A1- 2013 108 995
- US-A1- 2018 078 196
- US-A1- 2018 288 516
- US-A1- 2019 269 336

## Description

This application claims priority to U.S. Provisional Patent Application 62/990,182, filed March 16, 2020.

### TECHNICAL FIELD

This disclosure relates to hearing instruments.

### BACKGROUND

Hearing instruments are devices designed to be worn on, in, or near one or more of a user's ears. Common types of hearing instruments include hearing assistance devices (e.g., "hearing aids"), earbuds, headphones, hearables, cochlear implants, and so on. In some examples, a hearing instrument may be implanted or integrated into a user. Some hearing instruments include additional features beyond just environmental sound- amplification. For example, some modem hearing instruments include advanced audio processing for improved functionality, controlling and programming the hearing instruments, wireless communication with external devices including other hearing instruments (e.g., for streaming media), and so on.

US 2018/288516 A1 relates to ear buds having sensors to gather orientation information such as accelerometer measurements during user movements. A host electronic device may communicate wirelessly with the ear buds and may form part of an ear bud system that supplies the user with coaching and feedback while evaluating user performance of a head movement routine or other exercise routine. During operation, the ear buds may gather accelerometer data in a first reference frame such as a reference frame associated with the ear buds and may use a rotation matrix to rotate the data in the first reference frame into a second reference frame such as a neutral reference frame with a fixed orientation to the earth. The data in the neutral reference frame may be analyzed using a user head pose look-up table to categorize measured user head positions as corresponding to respective user head poses.

US 2019/269336 A1 relates to a wearable system wherein elements of the system, including various sensors adapted to detect biometric and other data and/or to deliver drugs, are positioned proximal to, on the ear or in the ear canal of a person.

US 2018/078196 A1 relates to systems and methods for assessing compliance with position therapy. In an embodiment, position therapy is provided to a user while the user is wearing a position therapy device. The position therapy comprises, by the device, collecting positional data, determining positions of the user over a time period based on the positional data, and, when it is determined that the user is in a target position, providing feedback to the user to influence the user to change to a non-target position.

### SUMMARY

The present invention provides a method with the features of claim 1, a system with the features of claim 7 and a computer-readable medium with the features of claim 14.

This disclosure describes techniques for detecting a posture of a user of one or more hearing instruments and determining whether the posture of the user is a target posture for the user. Example target activities include sitting, standing, walking, sleeping, and so on. A processing system may generate information about the posture of the user and provide the information to the user, another person, or one or more computing devices.

In one example, this disclosure describes a method comprising: obtaining, by a processing system, signals that are generated by or generated based on data from one or more sensors that are included in one or more hearing instruments; determining, by the processing system, based on the signals, whether a posture of a user of the one or more hearing instruments is a target posture for the user; and generating, by the processing system, information based on the posture of the user.

In another example, this disclosure describes a system comprising: one or more hearing instruments, wherein the one or more hearing instruments include sensors; a processing system comprising one or more processors implemented in circuitry, wherein the one or more processors are configured to: obtain signals that are generated by or generated based on data from the sensors; determine, based on the signals, whether a posture of a user of the one or more hearing instruments is a target posture of the user; and generate information based on the posture of the user.

This disclosure additionally describes a system comprising: means for obtaining signals that are generated by or generated based on data from one or more sensors that are included in one or more hearing instruments; means for determining, based on the signals, whether a posture of a user of the one or more hearing instruments is a target posture for the user; and means for generating information based on the posture of the user.

In another example, this disclosure describes a computer-readable medium comprising instructions stored thereon that, when executed, cause one or more processors to: obtain signals that are generated by or generated based on data from one or more sensors that are included in one or more hearing instruments; determine, based on the signals, whether a posture of a user of the one or more hearing instruments is a target posture for the user; and generate information based on the posture of the user.

The details of one or more aspects of the disclosure are set forth in the accompanying drawings and the description below. Other features, objects, and advantages of the techniques described in this disclosure will be apparent from the description, drawings, and claims.

### BRIEF DESCRIPTION OF DRAWINGS

FIG. 1 is a conceptual diagram illustrating an example system that includes one or more hearing instruments, in accordance with one or more techniques of this disclosure.
FIG. 2 is a block diagram illustrating example components of a hearing instrument, in accordance with one or more techniques of this disclosure.
FIG. 3 is a block diagram illustrating example components of a computing device, in accordance with one or more techniques of this disclosure.
FIG. 4 is a flowchart illustrating an example operation in accordance with one or more techniques described in this disclosure.
FIG. 5 is a block diagram illustrating example components of a hearing instrument and a computing device, in accordance with one or more techniques of this disclosure.
FIG. 6 is a block diagram illustrating example components of a hearing instrument, a computing device, and a wearable device, in accordance with one or more techniques of this disclosure.

### DETAILED DESCRIPTION

Poor posture is a common cause of musculoskeletal pain and other health problems. Poor posture often involves excess curvature of the thoracic and cervical spine. Such excess curvature may hinder breathing, impede circulation of blood or other internal fluids, cause pinched nerves, cause muscle stiffness, cause bone loss, cause headaches, and cause other medical conditions. Poor posture may also be a psychiatric indicator. Certain postures may be markers of aging, muscular dystrophy, Parkinson's disease, and camptocormia. In contrast, certain types of postures may be healthier. For example, a neutral spine posture may be a healthier spinal position for sitting or standing. In the neutral spine posture, the cervical spine is bent anteriorly, the thoracic spine is bent posteriorly, and the lumbar spine is bent anteriorly within specific ranges.

There are currently devices on the market for coaching users on proper posture. However, there are a number of problems associated with such devices. For instance, it is difficult for existing devices to discreetly provide information to users about their posture. For instance, users may not want other people to hear a device reminding the users to sit up straight. Similarly, providing haptic information causes audible sounds that may be disturbing or draw unwanted attention. Moreover, certain types of improper postures, such as forward jutting of the head, may be difficult to detect reliably using back-worn sensors or may be difficult to detect discreetly. Thus, posture detection and provision of information about the posture may be difficult or unreliable for such devices.

Techniques of this disclosure may address one or more of these problems. As described herein, a processing system obtains one or more signals that are generated by or generated based on data from one or more sensors that are included in one or more hearing instruments. The processing system determines, based on the signals,
whether a posture of a user of the one or more hearing instruments is a target posture for the user. In this disclosure, there may be different target postures for different activities. Additionally, the processing system may generate information about the posture of the user.

The use of sensors in hearing instruments may address one or more of the problems mentioned above because these sensors are essentially at stable positions relative to the user's head and therefore may be able to detect postures that are otherwise not detectable or reliably detectable. Moreover, hearing instruments may be able to provide discreet audio information to users that other people are not able to hear. The techniques of this disclosure may be especially advantageous because poor posture is especially a problem among older adults, who are also the most likely to use hearing instruments, such as hearing aids. Thus, it would be less surprising to see a hearing instrument worn by an older adult, even if that older adult does not have hearing loss that would otherwise cause the older adult to use a hearing aid, thereby potentially avoiding stigma associated with wearable a head-mounted sensor device.

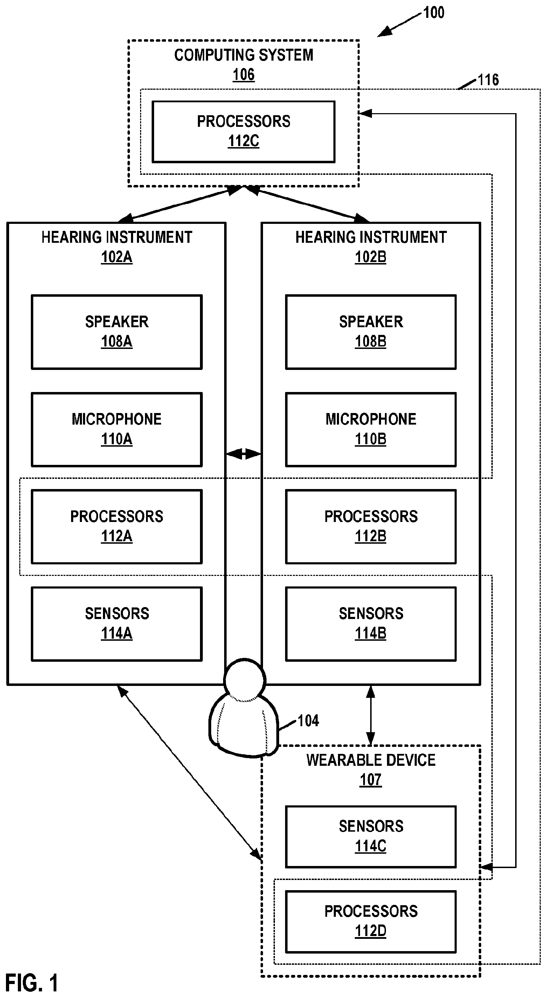

FIG. 1 is a conceptual diagram illustrating an example system 100 that includes hearing instruments 102A, 102B, in accordance with one or more techniques of this disclosure. This disclosure may refer to hearing instruments 102A and 102B collectively, as "hearing instruments 102." A user 104 may wear hearing instruments 102. In some instances, user 104 may wear a single hearing instrument. In other instances, user 104 may wear two hearing instruments, with one hearing instrument for each ear of user 104.

Hearing instruments 102 may include one or more of various types of devices that are configured to provide auditory stimuli to user 104 and that are designed for wear and/or implantation at, on, or near an ear of user 104. Hearing instruments 102 may be worn, at least partially, in the ear canal or concha. One or more of hearing instruments 102 may include behind the ear (BTE) components that are worn behind the ears of user 104. In some examples, hearing instruments 102 include devices that are at least partially implanted into or integrated with the skull of user 104. In some examples, one or more of hearing instruments 102 provides auditory stimuli to user 104 via a bone conduction pathway.

In any of the examples of this disclosure, each of hearing instruments 102 may include a hearing assistance device. Hearing assistance devices include devices that help a user hear sounds in the user's environment. Example types of hearing assistance devices may include hearing aid devices, Personal Sound Amplification Products (PSAPs), cochlear implant systems (which may include cochlear implant magnets, cochlear implant transducers, and cochlear implant processors), bone-anchored or osseointegrated hearing aids, and so on. In some examples, hearing instruments 102 are over-the-counter, direct-to-consumer, or prescription devices. Furthermore, in some examples, hearing instruments 102 include devices that provide auditory stimuli to user 104 that correspond to artificial sounds or sounds that are not naturally in the environment of user 104, such as recorded music, computer-generated sounds, or other types of sounds. For instance, hearing instruments 102 may include so-called "hearables," earbuds, earphones, or other types of devices that are worn on or near the ears of user 104. Some types of hearing instruments provide auditory stimuli to user 104 corresponding to sounds from the environment of user 104 and also artificial sounds.

In some examples, one or more of hearing instruments 102 includes a housing or shell that is designed to be worn in the ear for both aesthetic and functional reasons and encloses the electronic components of the hearing instrument. Such hearing instruments may be referred to as in-the-ear (ITE), in-the-canal (ITC), completely-in-the-canal (CIC), or invisible-in-the-canal (IIC) devices. In some examples, one or more of hearing instruments 102 may be behind-the-ear (BTE) devices, which include a housing worn behind the ear that contains all of the electronic components of the hearing instrument, including the receiver (e.g., a speaker). The receiver conducts sound to an earbud inside the ear via an audio tube. In some examples, one or more of hearing instruments 102 are receiver-in-canal (RIC) hearing-assistance devices, which include housings worn behind the ears that contain electronic components and housings worn in the ear canals that contain receivers.

Hearing instruments 102 may implement a variety of features that help user 104 hear better. For example, hearing instruments 102 may amplify the intensity of incoming sound, amplify the intensity of certain frequencies of the incoming sound, translate or compress frequencies of the incoming sound, and/or perform other functions to improve the hearing of user 104. In some examples, hearing instruments 102 implement a directional processing mode in which hearing instruments 102 selectively amplify sound originating from a particular direction (e.g., to the front of user 104) while potentially fully or partially canceling sound originating from other directions. In other words, a directional processing mode may selectively attenuate off-axis unwanted sounds. The directional processing mode may help user 104 understand conversations occurring in crowds or other noisy environments. In some examples, hearing instruments 102 use beamforming or directional processing cues to implement or augment directional processing modes.

In some examples, hearing instruments 102 reduce noise by canceling out or attenuating certain frequencies. Furthermore, in some examples, hearing instruments 102 may help user 104 enjoy audio media, such as music or sound components of visual media, by outputting sound based on audio data wirelessly transmitted to hearing instruments 102.

Hearing instruments 102 may be configured to communicate with each other. For instance, in any of the examples of this disclosure, hearing instruments 102 may communicate with each other using one or more wireless communication technologies. Example types of wireless communication technology include Near-Field Magnetic Induction (NFMI) technology, 900MHz technology, BLUETOOTH^{™} technology, WI-FI ^{™} technology, audible sound signals, ultrasonic communication technology, infrared communication technology, inductive communication technology, or other types of communication that do not rely on wires to transmit signals between devices. In some examples, hearing instruments 102 use a 2.4 GHz frequency band for wireless communication. In examples of this disclosure, hearing instruments 102 may communicate with each other via non-wireless communication links, such as via one or more cables, direct electrical contacts, and so on.

As shown in the example of FIG. 1, system 100 may also include a computing system 106. In other examples, system 100 does not include computing system 106. Computing system 106 includes one or more computing devices, each of which may include one or more processors. For instance, computing system 106 may include one or more mobile devices (e.g., smartphones, tablet computers, etc.), server devices, personal computer devices, handheld devices, wireless access points, smart speaker devices, smart televisions, medical alarm devices, smart key fobs, smartwatches, motion or presence sensor devices, smart displays, screen-enhanced smart speakers, wireless routers, wireless communication hubs, prosthetic devices, mobility devices, special-purpose devices, hearing instrument accessory devices, and/or other types of devices. Hearing instrument accessory devices may include devices that are configured specifically for use with hearing instruments 102. Example types of hearing instrument accessory devices may include charging cases for hearing instruments 102, storage cases for hearing instruments 102, media streamer devices, phone streamer devices, external microphone devices, remote controls for hearing instruments 102, and other types of devices specifically designed for use with hearing instruments 102.

Actions described in this disclosure as being performed by computing system 108 may be performed by one or more of the computing devices of computing system 108. One or more of hearing instruments 102 may communicate with computing system 108 using wireless or non-wireless communication links. For instance, hearing instruments 102 may communicate with computing system 108 using any of the example types of communication technologies described elsewhere in this disclosure.

Furthermore, as shown in the example of FIG. 1, system 100 may include a wearable device 107 separate from hearing instruments 102. Wearable device 107 may include one or more processors 112D. Furthermore, wearable device 107 may include one or more sensors 114C. Wearable device 107 may be configured to communicate with one or more of hearing instruments 102 and/or one or more devices in computing system 106. Wearable device 107 may include one of a variety of different types of devices. For example, wearable device 107 may be worn on a back of user 104. In this example, wearable device 107 may be held onto the back of user 104 with an adhesive, held in place by straps or a garment, or otherwise held in position on the back of user 104. In another example, wearable device 107 includes a pendant worn around a neck of user 104. In another example, wearable device 107 is worn on a neck or a shoulder of user 104.

In the example of FIG. 1, hearing instrument 102A includes a speaker 108A, a microphone 110A, one or more processors 112A, and one or more sensors 114A. Hearing instrument 102B includes a speaker 108B, a microphone 110B, one or more processors 112B, and one or more sensors 114B. This disclosure may refer to speaker 108A and speaker 108B collectively as "speakers 108." This disclosure may refer to microphone 110A and microphone 110B collectively as "microphones 110." This disclosure may refer to sensors 114A, sensors 114B, and sensors 114C collectively as "sensors 114." Computing system 106 includes one or more processors 112C. Processors 112C may be distributed among one or more devices of computing system 106. This disclosure may refer to processors 112A, 112B, 112C, and 112D collectively as "processors 112." Processors 112 may be implemented in circuitry and may include microprocessors, application-specific integrated circuits, digital signal processors, or other types of circuits.

As noted above, hearing instruments 102A, 102B, computing system 106, and wearable device 107 may be configured to communicate with one another. Accordingly, processors 112 may be configured to operate together as a processing system 116. Thus, discussion in this disclosure of actions performed by processing system 116 may be performed by one or more processors in one or more of hearing instrument 102A, hearing instrument 102B, computing system 106, or wearable device 107, either separately or in coordination. Moreover, it should be appreciated that processing system 116 does not need to include each of processors 112A, 112B, 112C, 112D. For instance, processing system 116 may be limited to processors 112A and not processors 112B, 112C, or 112D.

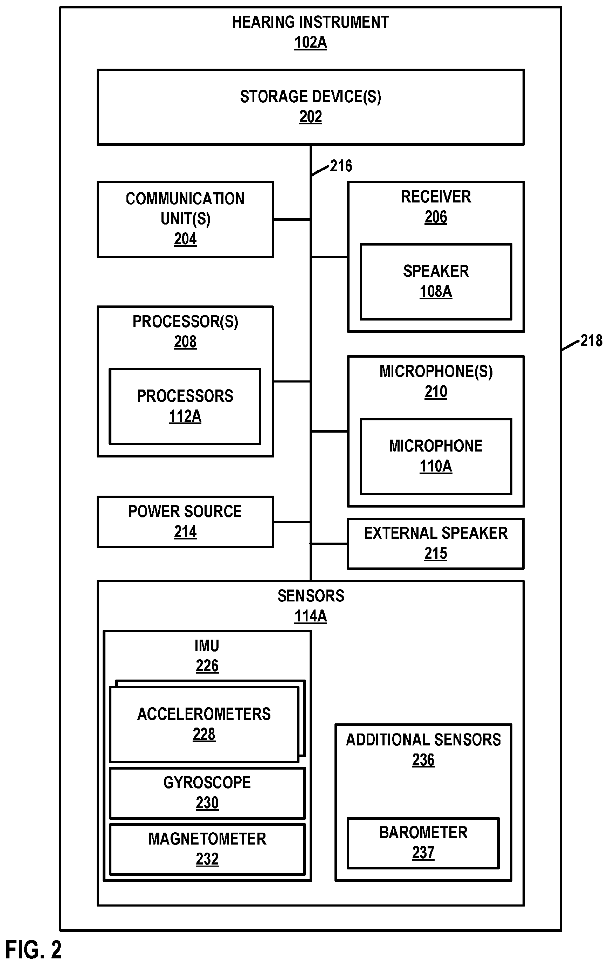

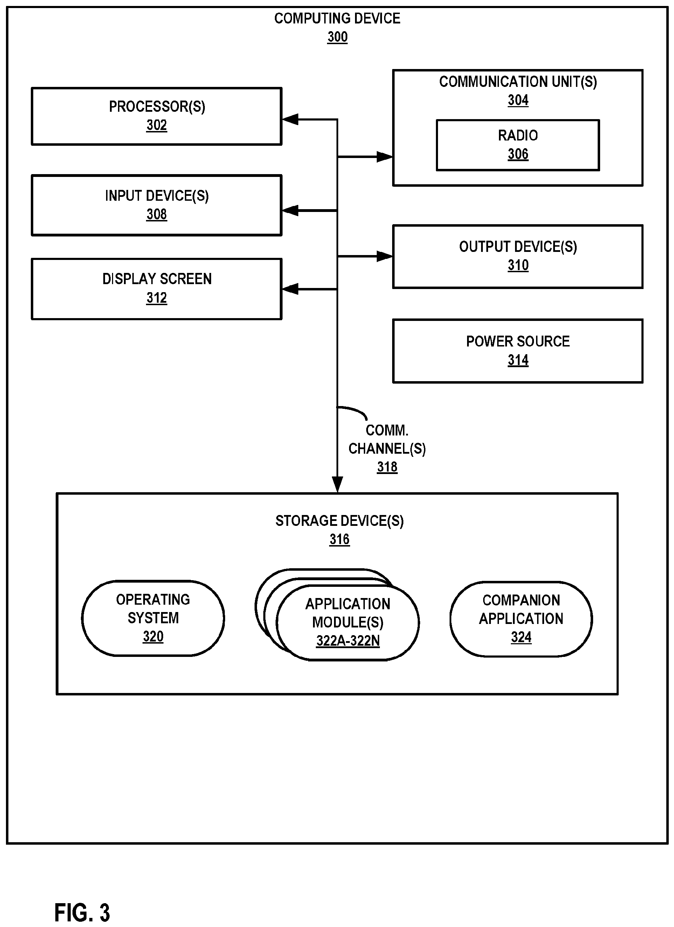

It will be appreciated that hearing instruments 102, computing system 106, and/or wearable device 107 may include components in addition to those shown in the example of FIG. 1, e.g., as shown in the examples of FIG. 2 and FIG. 3. For instance, each of hearing instruments 102 may include one or more additional microphones configured to detect sound in an environment of user 104. The additional microphones may include omnidirectional microphones, directional microphones, or other types of microphones.

As described herein, processing system 116 may obtain one or more signals that are generated by or generated based on data from sensors 114A, 114B that are included in one or more of hearing instruments 102A, 102B. Processing system 116 may determine, based on the signals, a posture of a user of the hearing instruments 102A, 102B. For example, processing system 116 may determine, based on the signals, whether a posture of user 104 is a target posture for user 104. Additionally, processing system 116 may generate information based on the posture of user 104. For instance, processing system 116 may generate information that reminds user 104 to adopt the target posture of the current posture of user 104 is not the target posture.

In some examples, processing system 116 may obtain one or more signals that are generated by wearable device 107. For instance, processing system 116 may obtain signals that are generated by or based on data generated by sensors 114C of wearable device 107. Processing system 116 may determine the posture of user 104 based on the signals generated by or generated based on data from sensors 114A, 114B, and based on the signals generated by or generated based on data from sensors 114C. Use of the signals generated by wearable device 107 may enhance the ability of processing system 116 to determine the posture of user 104.

FIG. 2 is a block diagram illustrating example components of hearing instrument 102A, in accordance with one or more aspects of this disclosure. Hearing instrument 102B may include the same or similar components of hearing instrument 102A shown in the example of FIG. 2. Thus, the discussion of FIG. 2 may apply with respect to hearing instrument 102B. In the example of FIG. 2, hearing instrument 102A includes one or more storage devices 202, one or more communication units 204, a receiver 206, one or more processors 208, one or more microphones 210, sensors 114A, a power source 214, an external speaker 215, and one or more communication channels 216. Communication channels 216 provide communication between storage devices 202, communication unit(s) 204, receiver 206, processor(s) 208, microphone(s) 210, sensors 114A, external speaker 215, and potentially other components of hearing instrument 102A. Components 202, 204, 206, 208, 210, 114A, 215, and 216 may draw electrical power from power source 214.

In the example of FIG. 2, each of components 202, 204, 206, 208, 210, 114A, 214, 215, and 216 are contained within a single housing 218. For instance, in examples where hearing instrument 102A is a BTE device, each of components 202, 204, 206, 208, 210, 114A, 214, 215, and 216 may be contained within a behind-the-ear housing. In examples where hearing instrument 102A is an ITE, ITC, CIC, or IIC device, each of components 202, 204, 206, 208, 210, 114A, 214, 215, and 216 may be contained within an in-ear housing. However, in other examples of this disclosure, components 202, 204, 206, 208, 210, 114A, 214, 215, and 216 are distributed among two or more housings. For instance, in an example where hearing instrument 102A is a RIC device, receiver 206, one or more of microphones 210, and one or more of sensors 114A may be included in an in-ear housing separate from a behind-the-ear housing that contains the remaining components of hearing instrument 102A. In such examples, a RIC cable may connect the two housings.

Furthermore, in the example of FIG. 2, sensors 114A include an inertial measurement unit (IMU) 226 that is configured to generate data regarding the motion of hearing instrument 102A. IMU 226 may include a set of sensors. For instance, in the example of FIG. 2, IMU 226 includes one or more accelerometers 228, a gyroscope 230, a magnetometer 232, combinations thereof, and/or other sensors for determining the motion of hearing instrument 102A. Furthermore, in the example of FIG. 2, hearing instrument 102A may include one or more additional sensors 236. Additional sensors 236 may include a photoplethysmography (PPG) sensor, blood oximetry sensors, blood pressure sensors, electrocardiograph (EKG) sensors, body temperature sensors, electroencephalography (EEG) sensors, environmental temperature sensors, environmental pressure sensors, environmental humidity sensors, skin galvanic response sensors, and/or other types of sensors. As shown in the example of FIG. 2, additional sensors 236 may include a barometer 237. In other examples, hearing instrument 102A and sensors 114A may include more, fewer, or different components. Processing system 116 (FIG. 1) may use signals by sensor 114A and/or data from sensors 114A to determine a posture of user 104.

Storage device(s) 202 may store data. Storage device(s) 202 may include volatile memory and may therefore not retain stored contents if powered off. Examples of volatile memories may include random access memories (RAM), dynamic random access memories (DRAM), static random access memories (SRAM), and other forms of volatile memories known in the art. Storage device(s) 202 may include non-volatile memory for long-term storage of information and may retain information after power on/off cycles. Examples of non-volatile memory may include flash memories or forms of electrically programmable memories (EPROM) or electrically erasable and programmable (EEPROM) memories.

Communication unit(s) 204 may enable hearing instrument 102A to send data to and receive data from one or more other devices, such as a device of computing system 106 (FIG. 1), another hearing instrument (e.g., hearing instrument 102B), a hearing instrument accessory device, a mobile device, wearable device 107 (FIG. 1), or other types of devices. Communication unit(s) 204 may enable hearing instrument 102A to use wireless or non-wireless communication technologies. For instance, communication unit(s) 204 enable hearing instrument 102A to communicate using one or more of various types of wireless technology, such as a BLUETOOTH^{™} technology, 3G, 4G, 4G LTE, 5G, ZigBee, WI-FI^{™}, Near-Field Magnetic Induction (NFMI), ultrasonic communication, infrared (IR) communication, or another wireless communication technology. In some examples, communication unit(s) 204 may enable hearing instrument 102A to communicate using a cable-based technology, such as a Universal Serial Bus (USB) technology.

Receiver 206 includes one or more speakers for generating audible sound. In the example of FIG. 2, receiver 206 includes speaker 108A (FIG. 1). The speakers of receiver 206 may generate sounds that include a range of frequencies. In some examples, the speakers of receiver 206 includes "woofers" and/or "tweeters" that provide additional frequency range. Receiver 206 may output audible information to user 104 about the posture of user 104. As shown in the example of FIG. 2, hearing instrument 102A may also include an external speaker 215 that is configured to generate sound that is not directed into an ear canal of user 104.

Processor(s) 208 include processing circuits configured to perform various processing activities. Processor(s) 208 may process signals generated by microphone(s) 210 to enhance, amplify, or cancel-out particular channels within the incoming sound. Processor(s) 208 may then cause receiver 206 to generate sound based on the processed signals. In some examples, processor(s) 208 include one or more digital signal processors (DSPs). In some examples, processor(s) 208 may cause communication unit(s) 204 to transmit one or more of various types of data. For example, processor(s) 208 may cause communication unit(s) 204 to transmit data to computing system 106. Furthermore, communication unit(s) 204 may receive audio data from computing system 106 and processor(s) 208 may cause receiver 206 to output sound based on the audio data. In the example of FIG. 2, processor(s) 208 include processors 112A (FIG. 1).

Microphone(s) 210 detect incoming sound and generate one or more electrical signals (e.g., an analog or digital electrical signal) representing the incoming sound. In the example of FIG. 2, microphones 210 include microphone 110A (FIG. 1). In some examples, microphone(s) 210 include directional and/or omnidirectional microphones.

FIG. 3 is a block diagram illustrating example components of computing device 300, in accordance with one or more aspects of this disclosure. FIG. 3 illustrates only one particular example of computing device 300, and many other example configurations of computing device 300 exist. Computing device 300 may be a computing device in computing system 106 (FIG. 1).

As shown in the example of FIG. 3, computing device 300 includes one or more processors 302, one or more communication units 304, one or more input devices 308, one or more output device(s) 310, a display screen 312, a power source 314, one or more storage device(s) 316, and one or more communication channels 318. Computing device 300 may include other components. For example, computing device 300 may include physical buttons, microphones, speakers, communication ports, and so on. Communication channel(s) 318 may interconnect each of components 302, 304, 308, 310, 312, and 316 for inter-component communications (physically, communicatively, and/or operatively). In some examples, communication channel(s) 318 may include a system bus, a network connection, an inter-process communication data structure, or any other method for communicating data. Power source 314 may provide electrical energy to components 302, 304, 308, 310, 312 and 316.

Storage device(s) 316 may store information required for use during operation of computing device 300. In some examples, storage device(s) 316 have the primary purpose of being a short-term and not a long-term computer-readable storage medium. Storage device(s) 316 may be volatile memory and may therefore not retain stored contents if powered off. In some examples, storage device(s) 316 includes non-volatile memory that is configured for long-term storage of information and for retaining information after power on/off cycles. In some examples, processor(s) 302 of computing device 300 may read and execute instructions stored by storage device(s) 316.

Computing device 300 may include one or more input devices 308 that computing device 300 uses to receive user input. Examples of user input include tactile, audio, and video user input. Input device(s) 308 may include presence-sensitive screens, touch-sensitive screens, mice, keyboards, voice responsive systems, microphones, motion sensors capable of detecting gestures (e.g., head nods or tapping), or other types of devices for detecting input from a human or machine.

Communication unit(s) 304 may enable computing device 300 to send data to and receive data from one or more other computing devices (e.g., via a communication network, such as a local area network or the Intemet). For instance, communication unit(s) 304 may be configured to receive data sent by hearing instrument(s) 102, receive data generated by user 104 of hearing instrument(s) 102, receive and send data, receive and send messages, and so on. In some examples, communication unit(s) 304 may include wireless transmitters and receivers that enable computing device 300 to communicate wirelessly with the other computing devices. For instance, in the example of FIG. 3, communication unit(s) 304 include a radio 306 that enables computing device 300 to communicate wirelessly with other computing devices, such as hearing instruments 102 (FIG. 1). Examples of communication unit(s) 304 may include network interface cards, Ethernet cards, optical transceivers, radio frequency transceivers, or other types of devices that are able to send and receive information. Other examples of such communication units may include BLUETOOTH^{™}, 3G, 4G, 5G, and WI-FI^{™} radios, Universal Serial Bus (USB) interfaces, etc. Computing device 300 may use communication unit(s) 304 to communicate with one or more hearing instruments (e.g., hearing instruments 102 (FIG. 1, FIG. 2)). Additionally, computing device 300 may use communication unit(s) 304 to communicate with one or more other devices.

Output device(s) 310 may generate output. Examples of output include tactile, audio, and video output. Output device(s) 310 may include presence-sensitive screens, sound cards, video graphics adapter cards, speakers, displays such as liquid crystal displays (LCD) or light emitting displays (LEDs), or other types of devices for generating output. Output device(s) 310 may include display screen 312. In some examples, output device(s) 310 may include virtual reality, augmented reality, or mixed reality display devices.

Processor(s) 302 may read instructions from storage device(s) 316 and may execute instructions stored by storage device(s) 316. Execution of the instructions by processor(s) 302 may configure or cause computing device 300 to provide at least some of the functionality ascribed in this disclosure to computing device 300 or components thereof (e.g., processor(s) 302). As shown in the example of FIG. 3, storage device(s) 316 include computer-readable instructions associated with operating system 320, application modules 322A-322N (collectively, "application modules 322"), and a companion application 324.

Execution of instructions associated with operating system 320 may cause computing device 300 to perform various functions to manage hardware resources of computing device 300 and to provide various common services for other computer programs. Execution of instructions associated with application modules 322 may cause computing device 300 to provide one or more of various applications (e.g., "apps," operating system applications, etc.). Application modules 322 may provide applications, such as text messaging (e.g., SMS) applications, instant messaging applications, email applications, social media applications, text composition applications, and so on.

Companion application 324 is an application that may be used to interact with hearing instruments 102, view information about hearing instruments 102, or perform other activities related to hearing instruments 102, thus serving as a companion to hearing instruments 102. Execution of instructions associated with companion application 324 by processor(s) 302 may cause computing device 300 to perform one or more of various functions. For example, execution of instructions associated with companion application 324 may cause computing device 300 to configure communication unit(s) 304 to receive data from hearing instruments 102 and use the received data to present data to a user, such as user 104 or a third-party user. In some examples, companion application 324 is an instance of a web application or server application. In some examples, such as examples where computing device 300 is a mobile device or other type of computing device, companion application 324 may be a native application.

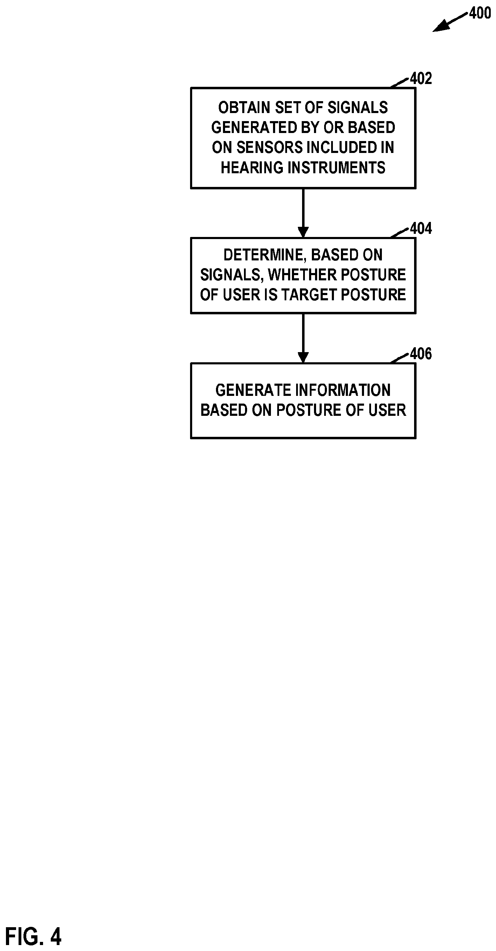

FIG. 4 is a flowchart illustrating an example operation 400 in accordance with one or more techniques of this disclosure. Other examples of this disclosure may include more, fewer, or different actions. In some examples, actions in the flowcharts of this disclosure may be performed in parallel or in different orders.

In the example of FIG. 4, processing system 116 may obtain signals that are generated by or generated based on data from one or more sensors that are included in hearing instruments 102 (402). As described elsewhere in this disclosure, hearing instruments 102 are configured to output sound. Signals generated based on data from the sensors may include data that includes features extracted from signals directly produced by the sensors or data otherwise generated by processing the signals produced by the sensors.

Furthermore, in the example of FIG. 4, processing system 116 may determine, based on the signals, whether a posture of user 104 of hearing instruments 102 is a target posture (404). The target posture may be for a specific activity, such as sitting or standing. In some examples, the target posture is a neutral spine posture. In some examples, the target posture is a posture that is intermediate between a preintervention posture of user 104 and the neutral spine posture. In other examples, the target posture may be a supine posture, e.g., when the activity is sleeping. The target posture may be established by a healthcare professional, may be a preset position, may be established by user 104, or may otherwise be established.

Processing system 116 may determine whether the posture of user 104 is the target posture for an activity in various ways. For instance, in some examples, processing system 116 may store net displacement values that includes a net displacement value for each degree of freedom in a plurality of degrees of freedom. The degrees of freedom may correspond to anterior/posterior movement, superior/inferior movement, lateral movement, roll, pitch, and yaw. The net displacement value for a degree of freedom indicates a net amount of displacement in a direction corresponding to the degree of freedom. For instance, the net displacement value for an anterior/posterior degree of freedom may indicate that hearing instrument 102A has moved 1.5 inches (3,81 cm) in the anterior direction. Additionally, in this example, processing system 116 performs a calibration process. As part of performing the calibration process, processing system 116 may reset the net displacement values based on receiving an indication (e.g., from user 104, a clinician, or other person) that user 104 has assumed the target posture. For instance, processing system 116 may reset each of the net displacement values to 0. Subsequently, processing system 116 may update the net displacement values based on the signals. Processing system 116 may determine that user 104 has the target posture based on the net displacement values. For instance, processing system 116 may determine that user 104 has the target posture based on each of the net displacement values being within a respective predefined range of target values corresponding to the target posture. The respective predefined range is a range of net displacement values that may be consistent with user 104 having the target posture.

Furthermore, in some examples where processing system 116 stores and uses net displacement values, processing system 116 may analyze the signals to identify segments of the signals corresponding to posture-related movements that are distinct from walking or other locomotion-related movements. For instance, processing system 116 may include a machine-learning model (e.g., an artificial neural network, etc.) that classifies segments of the signals as being associated with posture-related movements. Processing system 116 may update the net displacement values based only on segments of the signals that are associated with posture-related movements. This may allow processing system 116 to ensure that the net displacement values reflect the net displacement of hearing instruments 102 attributable to changes in the posture of user 104, not overall displacement of user 104.

In some examples, to determine whether the posture of user 104 is the target posture, processing system 116 may determine, based on the signals, a current direction of gravity. For instance, in this example, the sensors may include one or more accelerometers (e.g., accelerometers 228 of IMU 226) configured to detect acceleration caused by Earth's gravity. Furthermore, in this example, processing system 116 may perform a calibration process. As part of performing the calibration process, processing system 116 may establish, based on receiving an indication that user 104 has assumed the target posture (e.g., a voice command, a tapping input to one or more of hearing instruments 102, a command to a mobile device, etc.), a gravity bias value for the target posture based on the current direction of gravity. The gravity bias value may indicate an angle between a predetermined axis of hearing instrument 102A and a direction of gravitational acceleration. Establishing the gravity bias value for the target posture allows processing system 116 to determine what gravity bias value corresponds to the target posture. In this example, after calibrating the gravity bias value, processing system 116 updates a current gravity bias value based on subsequent information in the signals. Thus, as user 104 subsequently moves around, processing system 116 may update the current gravity bias value so that the gravity bias value continues to indicate the current angle between the predetermined axis of hearing instrument 102A and the direction of gravitational acceleration. In this example, processing system 116 may determine whether the posture of user 104 is the target posture based on the gravity bias value for the target posture and the current gravity bias value. For example, processing system 116 may determine that the posture of user 104 is the target posture when the gravity bias value is consistent with the target posture (e.g., the current gravity bias value is within a range of the gravity bias value recorded during calibration). For instance, user 104 may not have the target posture (e.g., user 104 may have a poor posture) if an anterior tilt of the head of user 104 is too large. In some examples, determining that the gravity bias value is consistent with the target posture may be a necessary but not sufficient condition for determining that user 104 has the target posture. For instance, user 104 might not have the target posture if the head of user 104 is thrust forward but is held level.

In some examples, the sensors include one or more microphones (e.g., microphones 210) and one or more of hearing instruments 102 include a speaker (e.g., external speaker 215). In such examples, processing system 116 may cause the speaker to periodically emit a sound, such as an ultrasonic or subsonic sound. The signals may include one or more audio signals detected by the microphones. Furthermore, in this example, processing system 116 may obtain information, via the microphones, indicating reflections of the sound emitted by the speaker in the one or more audio signals, e.g., by sending a signal to start detection of sound. During a calibration process, processing system 116 may instruct user 104 to assume the target posture and, in response to receiving an indication that user 104 has assumed the target posture, processing system 116 may cause the speaker to emit the sound and determine a delay of reflections of the sound detected by the microphones. The delay may be considered a delay for the target posture. Processing system 116 may determine whether user 104 has the target posture based in part on a delay of the detected reflections of subsequently sounds emitted by the speaker. For instance, processing system 116 may compare a current delay to the delay for the target posture to determine whether the head of user 104 has moved away from the target posture. In this example, the sound may reflect off horizontal surfaces (e.g., the floor or ceiling). If the head of user 104 has moved downward (e.g., due to poor posture), the delay of the detected reflections decreases. If the head of user 104 has moved upward, the delay of the detected reflections increases.

In some examples, the sensors include a barometer (e.g., barometer 237 (FIG. 2)) and the signals include a signal from the barometer. In this example, processing system 116 may perform a calibration process. As part of performing the calibration process, processing system 116 may determine, based on receiving an indication that user 104 has assumed the target posture, a target altitude value based on the signal from the barometer. Altitude values may be in terms of barometric pressure, height above sea level, height relative to another level (e.g., a previous level), direction of altitude movement over time, or otherwise expressed. The target altitude value corresponds to an altitude of hearing instrument 102A when user 104 is in the target posture. In this example, processing system 116 may determine, based on the signal from the barometer, an altitude of the one or more hearing instruments. Furthermore, in this example, processing system 116 may determine whether the posture of user 104 is the target posture based in part on the altitude of the one or more hearing instruments. For instance, the head of user 104 is typically lower when user 104 is not in the target posture. Thus, if the current altitude of the barometer is less than the target altitude value by at least a specific amount, processing system 116 may determine that user 104 does not have the target posture. In some examples, processing system 116 may obtain satellite-based navigation information (e.g., Global Positioning System (GPS) information) for one or more of hearing instruments 102. In such examples, processing system 116 may use the satellite-based navigation information to establish a target altitude value when user 104 is in the target posture and may use later satellite-based navigation information for one or more of hearing instruments 102 to determine a current altitude. Processing system 116 may then use the current altitude and target altitude value as described above.

In some examples, user 104 has a secondary device equipped with instruments for detecting altitude (e.g., a barometer, a satellite-based navigation system, etc.) that is separate from hearing instruments 102 (and in some examples, separate from wearable device 107). For instance, the secondary device may be a mobile phone or other type of device that is typically carried near the waist of user 104. Processing system 116 may establish target altitude values based on data from hearing instruments 102 and also the secondary device. Processing system 116 may later obtain current altitude values for hearing instruments 102 and the secondary device. Processing system 116 may determine a difference between the current altitude values. If the difference between the current altitude values is less than a threshold based on the difference between the target altitude values (e.g., the difference between the target altitude values minus x percent), processing system 116 may determine that user 104 does not have the target posture.

In some examples, as part of determining whether the posture of user 104 is the target posture, processing system 116 may determine, based on one or more of the signals, whether a walking gait of user 104 is consistent with a camptocormia posture. In this example, processing system 116 may determine that user 104 does not have the target posture based on the walking gait of user 104 being consistent with the camptocormia posture. Processing system 116 may determine whether the walking gait of user 104 is consistent with the camptocormia posture based on IMU signals (e.g., signals from one or more IMUs of hearing instruments 102) in the signals. When user 104 is walking in the camptocormia posture, the IMU signals will indicate a forward-backward swaying motion in the walking gait of user 104. Thus, processing system 116 may determine that user 104 has the camptocormia posture when the IMU signals indicate that the forward-backward swaying motion in the walking gait of user 104 exceeds a threshold level.

In some examples, the signals generated by or generated based on data from sensors in hearing instruments 102 may be considered first signals and processing system 116 may obtain one or more second signals that are generated by wearable device 107, which are separate from the one or more hearing instruments 102. Processing system 116 may determine whether user 104 has the target posture based on the first signals and the second signals.

The second signals may include one or more types of information. For example, wearable device 107 may include a device that is worn on an upper back of user 104. In this example, wearable device 107 may include an IMU or other sensors to determine a gravity bias value for wearable device 107. The second signals may include the gravity bias value for wearable device 107. By comparing the gravity bias value for wearable device 107 and a gravity bias value determined based on sensors in hearing instruments 102 to corresponding gravity bias values determined during a calibration process, processing system 116 may determine whether a posture of user 104 is the target posture.

In some examples, wearable device 107 may be worn on a back of user 104 and wearable device 107 may generate a wireless signal detected by one or more of hearing instruments 102. The second signals may include the wireless signal. One or more characteristics of the wireless signal may change based on a distance between wearable device 107 and hearing instruments 102. For instance, if wearable device 107 is worn on the thoracic spine of user 104, the distance between wearable device 107 and hearing instruments 102 may be minimized when hearing instruments 102 are directly superior to wearable device 107 and may increase as the head of user 104 moves anteriorly relative to wearable device 107, as is typical when user 104 is slouching, thus the amplitude of the wireless signal may decrease when user 104 is slouching or a phase delay of the wireless signal as detected by hearing instruments 102 may be different depending on the distance of wearable device 107 from hearing instruments 102. In another example, if user 104 is slouching, an amplitude of the wireless signal may be more attenuated than when user 104 is not slouching because the wireless signal may pass through or around more of the body of user 104 when the head of user 104 is held further in front of the shoulders of user 104. Thus, in any of these examples, as part of determining whether the posture of user 104 is the target posture, processing system 116 may determine, based on one or more characteristics of the wireless signal, whether the posture of user 104 is the target posture.

In another examples, wearable device 107 includes a pendant worn around a neck of user 104. In this example, if user 104 is slouched forward, the pendant may swing in an anterior/posterior direction. However, if user 104 is sitting or walking with a neutral spine posture, the pendant may rest against the chest of user 104 or a swing of the pendant is interrupted by the chest or neck of user 104. Accordingly, in this example, wearable device 107 may include an IMU or other types of sensors to detect swinging of the pendant. Processing system 116 may obtain signals generated by sensors of wearable device 107 and determine, based at least in part on these signals, whether the posture of user 104 is the target posture. For instance, processing system 116 may determine that user 104 is not in the target posture if the signals generated by the IMU of wearable device 107 indicate an uninterrupted swinging motion of the pendant.

In the examples of this disclosure that involve a calibration process, such calibration processes may occur multiple times. For instance, a calibration process may occur each time sensor device 107 is put on user 104. In some examples, the calibration process may be performed on a periodic basis (e.g., daily, weekly, etc.). In some examples, the calibration process may occur when processing system 104 determines that user 104 has changed activities.

In some examples, processing system 116 may obtain signals from multiple wearable devices in addition to hearing instruments 102. For example, processing system 116 may obtain signals from one or more wearable devices worn on the back, chest, or shoulders of user 104 and/or obtain signals from a wearable device that includes a neck-worn pendant, e.g., as described elsewhere in this disclosure, or other wearable device.

In some examples, processing system 116 may determine whether user 104 is in a target posture based on multiple factors. For example, processing system 116 may determine based on a gravity bias value whether the current posture of user 104 is inconsistent with the target posture. Furthermore, in this example, processing system 116 may determine based on net displacement values whether the current posture of user 104 is the target posture. In this example, processing system 116 may make the determination that the posture of user 104 is the target posture if both of these two factors indicate that the posture of user 104 is the target posture and may determine that the posture of user 104 is not the target posture if either or both of these two factors indicate that the posture of user 104 is not the target posture.

In some examples, user 104 may have different target postures for different activities, such as sitting, standing, walking, playing a sport, and so on. Processing system 116 may automatically identify the activity of user 104 based on signals generated by or generated based on data from sensors in one or more of hearing instruments 102 and/or wearable device 107. Processing system 116 may determine whether the posture of user 104 is the target posture for the detected activity. In some examples, processing system 116 may receive an indication of user input specifying the activity.

Processing system 116 may generate information based on the posture of user 104 (406). Generating information may include calculating, selecting, determining, or arriving at the information. Processing system 116 may generate the information based on the posture of user 104 in various ways. For instance, in one example, processing system 116 may generate information that causes one or more of hearing instruments 102 to output auditory information regarding the posture of user 104 to user 104 while user 104 is using hearing instruments 102. In this example, because hearing instruments 102 provide the auditory information to user 104, typically only user 104 is able to hear the auditory information. Thus, people around user 104 are not aware of the auditory information to user 104 and the auditory information is therefore more discreet. The auditory information may instruct user 104 to assume a target posture. In some examples, the auditory information may include information about whether user 104 has met posture goals for user 104. In some examples, the auditory information may include information about how much time user 104 should spend in the target posture an upcoming time period (e.g., 1 hour) in order to meet the posture goals for user 104. The auditory information may take the form of verbal information or non-verbal audible cues, such as beeps or tones.

In some examples, processing system 116 may generate an email message or other type of message, such as an app-based notification or text message, containing the information based on the posture of user 104. In such examples, the message may include statistical information about the posture of user 104. For instance, the statistical information may include information about an amount of time user 104 spent in a target posture versus an amount of time user 104 was not in the target posture.

In some examples, processing system 116 may provide the generated information to one or more computing devices. For example, processing system 116 may provide the generated information to a computing device that uses the information for statistical analysis or scientific research.

In some examples, generating the information based on the posture of user 104 may involve generating feedback to user 104 about whether the posture of user 104 is the target posture. For instance, the feedback may inform user 104 that user 104 is or is not currently in the target posture.

Furthermore, in some examples, the target posture is a target posture for sleeping. In this example, processing system 116 may determine whether user 104 is asleep. Processing system 116 may determine whether user 104 is asleep in one or more of a variety of ways. For instance, in one example, processing system 116 may obtain signals (e.g., from sensors, such as sensors of IMU 226, inward-facing microphones, and/or other types of sensors, in hearing instruments 102) regarding a respiration pattern of user 104. In this example, processing system 116 may determine that user 104 is asleep based on the respiration pattern of user 104 being consistent with user 104 being asleep. In some examples, processing system 116 may obtain EEG signals (e.g., from EEG sensors included in one or more of hearing instruments 102). Processing system 116 may determine that user 104 is asleep based on the EEG signals. In some examples, processing system 116 may implement an artificial neural network or other machine-learning system that determines whether the obtained signals indicate that user 104 is asleep.

In addition to determining whether user 104 is asleep, processing system 116 may also determine whether user 104 is in a target posture for sleep. For instance, sleeping in a sitting posture may be associated with muscle stiffness, neck pain, shoulder pain, and other symptoms. Furthermore, sleeping in a prone posture (e.g., as opposed to a supine posture), or vice versa, may be associated with breathing difficulties or snoring. Accelerometers in hearing instruments 102 may differentiate the supine, prone, and upright postures based on directions of gravitational bias. In accordance with one or more techniques of this disclosure, processing system 116 may generate the information based on the posture of user 104 based on user 104 being asleep and not having the target posture for sleeping.

For example, if processing system 116 determines that user 104 is sleeping and is not in the target posture for sleep, processing system 116 may cause one or more of hearing instruments 102 to output audio data encouraging user 104 to change to the target posture for sleep. In some examples, if processing system 116 determines that user 104 is sleeping and is not in the target posture for sleep, processing system 116 may activate one or more haptic information units in one or more of hearing instruments 102 to encourage user 104 to change to the target posture for sleep. In some examples, if processing system 116 determines that user 104 is sleeping and user 104 is not in the target posture for sleep, processing system 116 may output a command to an adjustable bed (e.g., adjustable mechanically or by air) used by user 104 to elevate or lower a portion of the bed in a way that promotes the target posture (e.g., elevating a head portion of the bed may cause user 104 to shift out of a prone posture and into a supine posture). In a similar example, if processing system 116 determines that user 104 is sleeping in an upright position in a piece of adjustable furniture (e.g., an adjustable chair, bed, hospital bed), processing system 116 may automatically output a command to the piece of adjustable furniture to gently lower a portion of the piece of adjustable furniture to a more appropriate sleeping position.

In some examples, if processing system 116 determines that user 104 is sleeping and user 104 is not in the target posture for sleep, processing system 116 may generate a message (e.g., email message, notification message, etc.) informing user 104 of the benefit of sleeping in the target posture for sleep, informing user 104 of various statistics regarding user 104 sleeping in or not in the target posture for sleep, and so on. Determining whether user 104 is in a target posture for sleep using sensors in hearing devices may be especially advantageous in examples where the hearing devices are ITE, CIC, or IIC devices because such devices might be less disruptive to the sleep of user 104 than other types of sensor devices.

In some examples where the information generated by processing system 116 is provided to one or more people other than user 104, such information may be used to determine whether a medical intervention, such as a physical or mental wellness check, may be desirable. For example, development of poor posture may be a sign of depression. Accordingly, in this example, a healthcare provider or family member receiving information indicating that user 104 has developed poor (e.g., non-target posture) may want to check in on the mental wellness of user 104. Similarly, camptocormia or other disease in user 104 may develop over time. The information generated by processing system 116 may be evaluated by a healthcare provider or other person who may then arrange a physical exam of user 104 to check whether user 104 has developed camptocormia or other disease. In another example, if user 104 complains of headaches, muscle aches, neck soreness, or other associated problems, a clinician or other type of person may review the information about the posture of user 104 generated by processing system 116 to determine whether the posture of user 104 may be a factor in development of such problems. In some such examples, processing system 116 does not necessarily compare the posture of user 104 to a target posture. Rather, in some such examples, the information generated by processing system 116 about the posture of user 104 may include other types of information, such as a head angle relative to the thoracic or cervical spine of user 104, etc. In some examples, the target posture may be considered to be a reference posture instead of a posture that user 104 is attempting to attain.

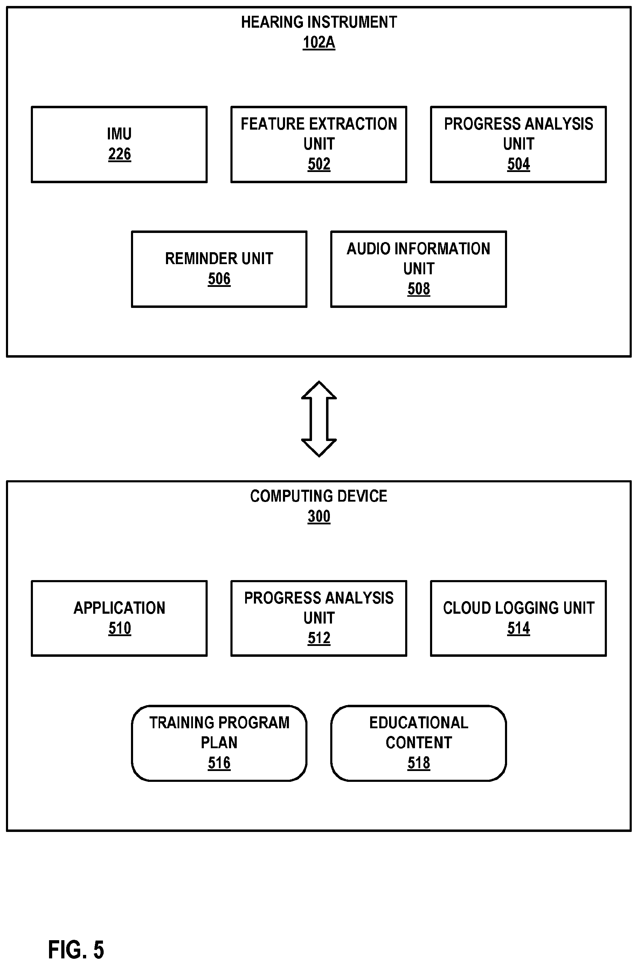

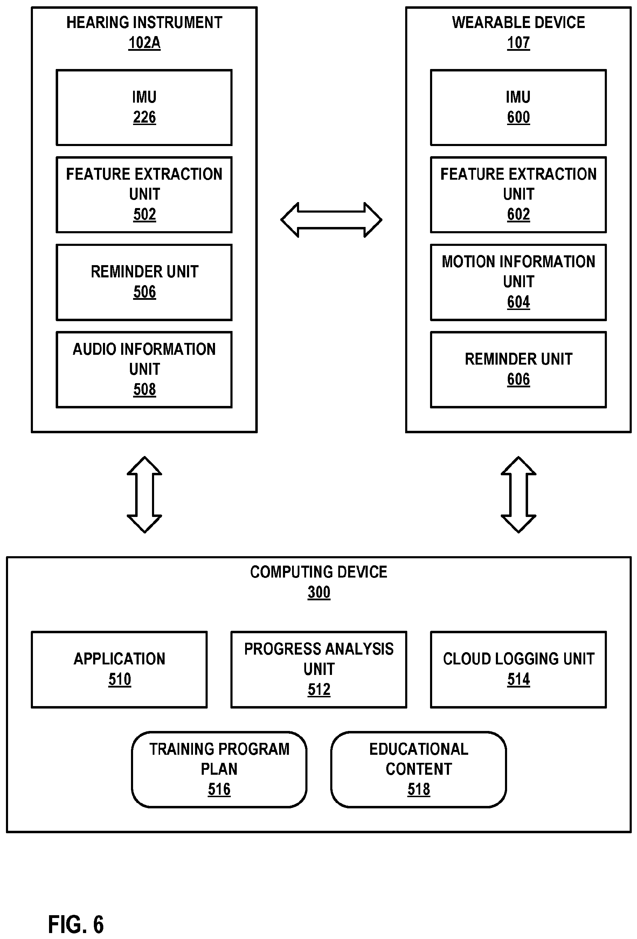

FIG. 5 is a block diagram illustrating example components of hearing instrument 102A and computing device 300, in accordance with one or more techniques of this disclosure. FIG. 6 is a block diagram illustrating example components of hearing instrument 102A, computing device 300, and wearable device 107, in accordance with one or more techniques of this disclosure. FIG. 5 and FIG. 6 are provided to illustrate example locations of sensors and examples of how functionality of processing system 116 may be distributed among hearing instruments 102, computing system 106, and wearable device 107. Although FIG. 5 and FIG. 6 are described with respect to hearing instrument 102A such description may apply to hearing instrument 102B, or functionality ascribed in this disclosure to hearing instrument 102A may be distributed between hearing instrument 102A and hearing instrument 102B.

In the example of FIG. 5, computing device 500 may be a computing device in computing system 106 (FIG. 1). Furthermore, in the example of FIG. 5, hearing instrument 102A includes IMU 226, a feature extraction unit 502, a progress analysis unit 504, a reminder unit 506, and an audio information unit 508. Computing device 500 includes an application 510, a progress analysis unit 512, a cloud logging unit 514, a training program plan 516, and educational content 518.

In the example of FIG. 5, feature extraction unit 502 may obtain signals, e.g., from IMU 226, and extract features from the signals. The extracted features may include data indicating relevant aspects of the signals. For example, feature extraction unit 502 may determine a current gravity bias value or current net displacement values based on signals from IMU 226. In such examples, hearing instrument 102A may transmit the extracted features to computing device 500.

Application 510 of computing device 500 may be one of application modules 322 (FIG. 3) or companion application 324 (FIG. 3). Application 510 of computing device 500 may determine, based on the extracted features and/or other data, whether a posture of user 104 is a target posture for an activity of user 104 (e.g., sitting, standing, sleeping, etc.). Application 510 may also generate information based on the posture of user 104. Generating the information may include causing audio information unit 508 of hearing instrument 102A to output audible information to user 104 while user 104 is using hearing instrument 102A.

Progress analysis unit 504 of hearing instrument 102A and/or progress analysis unit 512 of computing device 300 may determine whether user 104 is progressing toward a posture goal for user 104. User 104 or another person may set the posture goal for user 104. The posture goal may specify a desired amount or percentage of time during a time period (e.g., an hour, day, week, etc.) in which user 104 is to have a target posture, such as a neutral spine posture. In some instances, the target posture may be a posture that is not a neutral spine posture but is an improved posture relative to a previous posture assumed by user 104. Because long periods of poor posture may result in weakened muscles or ingrained habits, it may not be comfortable or reasonable for user 104 to maintain the target posture at all times. Rather, user 104 may need to work toward the posture goal for user 104 over time.

Reminder unit 506 of hearing instrument 102A may provide automated reminders to user 104 to assume the target posture. In some examples, reminder unit 506 may provide reminders to user 104 using audio information unit 508. Reminder unit 506 may provide reminders to user 104 on a periodic basis. In some examples, reminder unit 104 may provide reminders to user 104 on an event-driven basis. For instance, reminder unit 506 may provide reminders to user 104 when user 104 transitions from a sitting posture to a standing posture, or vice versa.

In examples where computing device 300 is a mobile device of user 104, such as a mobile phone, a hearing instrument accessory device for hearing instruments 102A, or another type of device close to hearing instruments 102, cloud logging unit 514 may upload data regarding the posture of user 104 to a cloud-based computing data, e.g., for storage and/or further processing.

In the example of FIG. 5, computing device 300 may store one or more of a training program plan 516 and educational content 518. Training program plan 516 may include data, such as text, images, and/or videos, that describe a plan for user 104 to improve the posture of user 104. Training program plan 516 may include data that is specific to user 104. Educational content 518 may include content to educate user 104 or other people about posture. In some examples, education content 518 is not specific to user 104. Computing device 300 may output training program plan 516 and/or educational content 518 for display (e.g., on display screen 312 (FIG. 3)) and/or provide audio output (e.g., using one or more of output devices 310) (FIG. 3)) based on training program plan 516 and/or educational content 518. In some examples, computing device 300 may cause hearing instruments 102 to output audio based on training program plan 516 and/or educational content 518.

In the example of FIG. 6, hearing instrument 102A (or hearing instrument 102B) includes IMU 226, feature extraction unit 502, reminder unit 506, audio information unit 508. Furthermore, in the example of FIG. 6, computing device 300 includes application 510, progress analysis unit 512, cloud logging unit 514, training program plan 516, and educational content 518. IMU 226, feature extraction unit 502, reminder unit 506, audio information unit 508, application 510, progress analysis unit 512, cloud logging unit 514, training program plan 516, and educational content 518 may have the same roles and functions as described above with respect to FIG. 5.

However, in the example of FIG. 6, wearable device 107 includes an IMU 600, a feature extraction unit 602, a motion information unit 604, and a reminder unit 606. IMU 600 may detect motion of wearable device 107. IMU 600 may be implemented in a similar way as IMU 226 (FIG. 2). Like feature extraction unit 502, feature extraction unit 602 may extract features from signals from IMU 600 and/or other sensors. Feature extraction unit 602 may transmit the extracted features to hearing instrument 102A or computing device 300. In some examples, wearable device 107 may receive features extracted by feature extraction unit 502 from hearing instrument 102A. In some such examples, wearable device 107 may determine based in part on the received features and extracted features from signals of sensors in wearable device 107 (e.g., IMU 600) whether a posture of user 104 is a target posture.

Motion information unit 604 of wearable device 107 may detect if user 104 is static or in transit (e.g., walking or running) to adjust the analysis accordingly. In some examples, if user 104 is in transit, application 510 does not determine whether the posture of user 104 is the target posture. In some examples, different target postures may be established for user 104 for times when user 104 is in transit and times when user 104 is not in transit. In such examples, application 510 may evaluate the posture of user 104 using the appropriate target posture for whether user 104 is static or in transit.

Furthermore, in the example of FIG. 6, reminder unit 606 of wearable device 107 may provide reminders to user 104 about the posture of user 104. In some examples, reminder unit 606 may provide the reminders to user 104 instead of reminder unit 506. In some examples, reminder unit 606 may provide audible or haptic reminders about the posture of user 104.

In this disclosure, ordinal terms such as "first," "second," "third," and so on, are not necessarily indicators of positions within an order, but rather may be used to distinguish different instances of the same thing. Furthermore, with respect to examples that involve personal data regarding a user, it may be required that such personal data only be used with the permission of the user.

In one or more examples, the functions described may be implemented in hardware, software, firmware, or any combination thereof. If implemented in software, the functions may be stored on or transmitted over, as one or more instructions or code, a computer-readable medium and executed by a hardware-based processing unit. Computer-readable media may include computer-readable storage media, which corresponds to a tangible medium such as data storage media, or communication media including any medium that facilitates transfer of a computer program from one place to another, e.g., according to a communication protocol. In this manner, computer-readable media generally may correspond to (1) tangible computer-readable storage media which is non-transitory or (2) a communication medium such as a signal or carrier wave. Data storage media may be any available media that can be accessed by one or more computers or one or more processing circuits to retrieve instructions, code and/or data structures for implementation of the techniques described in this disclosure. A computer program product may include a computer-readable medium.

By way of example, and not limitation, such computer-readable storage media may include RAM, ROM, EEPROM, CD-ROM or other optical disk storage, magnetic disk storage, or other magnetic storage devices, flash memory, cache memory, or any other medium that can be used to store desired program code in the form of instructions or store data structures and that can be accessed by a computer. Also, any connection is properly termed a computer-readable medium. For example, if instructions are transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. It should be understood, however, that computer-readable storage media and data storage media do not include connections, carrier waves, signals, or other transient media, but are instead directed to non-transient, tangible storage media. Disk and disc, as used herein, includes compact disc (CD), laser disc, optical disc, digital versatile disc (DVD), and Blu-ray disc, where disks usually reproduce data magnetically, while discs reproduce data optically with lasers. Combinations of the above should also be included within the scope of computer-readable media.

Functionality described in this disclosure may be performed by fixed function and/or programmable processing circuitry. For instance, instructions may be executed by fixed function and/or programmable processing circuitry. Such processing circuitry may include one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor," as used herein may refer to any of the foregoing structure or any other structure suitable for implementation of the techniques described herein. In addition, in some aspects, the functionality described herein may be provided within dedicated hardware and/or software modules. Also, the techniques could be fully implemented in one or more circuits or logic elements. Processing circuits may be coupled to other components in various ways. For example, a processing circuit may be coupled to other components via an internal device interconnect, a wired or wireless network connection, or another communication medium.

The techniques of this disclosure may be implemented in a wide variety of devices or apparatuses, an integrated circuit (IC) or a set of ICs (e.g., a chip set). Various components, modules, or units are described in this disclosure to emphasize functional aspects of devices configured to perform the disclosed techniques, but do not necessarily require realization by different hardware units. Rather, as described above, various units may be combined in a hardware unit or provided by a collection of interoperative hardware units, including one or more processors as described above, in conjunction with suitable software and/or firmware.

Various examples have been described. These and other examples are within the scope of the following claims.

## Claims

1. A method comprising:
obtaining, by a processing system (116), signals that are generated by or generated based on data from one or more sensors (114) that are included in one or more hearing instruments (102);
storing, by the processing system (116), net displacement values that include a net displacement value for each degree of freedom in a plurality of degrees of freedom,
wherein the net displacement values are derived based on signals generated by inertial measurement units, IMUs, comprised within the one or more sensors of the hearing instruments;
performing a calibration process, wherein performing the calibration process comprises, based on receiving an indication that the user (104) of the one or more hearing instruments has assumed a target posture, resetting, by the processing system (116), the net displacement values; and
updating, by the processing system (116), the net displacement values based on the signals;
wherein the method further comprises:
determining whether the posture of the user (104) is the target posture based on each of the net displacement values being within a respective predefined range;
determining, by the processing system (116), whether the user (104) is asleep and
whether the user (104) is in a target posture for sleep, wherein accelerometers comprised within the one or more sensors of the hearing instruments (102) differentiate supine, prone, and upright postures based on directions of gravitational bias; and
generating, by the processing system (116), information based on the user (104) being asleep and not having a target posture for sleeping.

2. The method of claim 1, wherein:
the method further comprises:
determining, by the processing system (116), based on the signals from the one or more accelerometers, a current direction of gravity;
based on receiving the indication that the user (104) has assumed the target posture, establishing, by the processing system (116), the gravitational bias value for the target posture based on the current direction of gravity; and
after establishing the gravitational bias value for the target posture, updating, by the processing system (116), a current gravitational bias value based on subsequent information in the signals, and
determining whether the posture of the user (104) is the target posture comprises determining, by the processing system (116), based on the current gravitational bias value and the gravitational bias value for the target posture, whether the posture of the user (104) is the target posture; and/or
wherein:
the sensors (114) include one or more microphones (110),
one or more of the one or more hearing instruments (102) includes a speaker (108),
the method further comprises:
causing, by the processing system (116), the speaker (108) to periodically emit a sound, wherein the signals include one or more audio signals detected by the one or more microphones (110);
obtaining information, by the processing system (116) via the microphones (110), indicating reflections of the sound emitted by the speaker (108) in the one or more audio signals; and
determining, by the processing system (116), whether the posture of the user (104) is the target posture based in part on a delay of the reflections of the sound; and/or
wherein:
the sensors (114) include a barometer (237),
the signals include a signal from the barometer (237), and
the method further comprises:
based on receiving the indication that the user (104) has assumed the target posture, determining, by the processing system (116), a target altitude value based on the signal from the barometer (237);
determining, by the processing system (116), based on the signal from the barometer (237), an altitude of the one or more hearing instruments (102); and
determining, by the processing system (116), whether the posture of the user (104) is the target posture based in part on the altitude of the one or more hearing instruments (102); and/or
wherein determining whether the posture of the user (104) is the target posture further comprises:
determining, by the processing system (116), based on one or more of the signals, whether a walking gait of the user (104) is consistent with a camptocormia posture; and
determining, by the processing system (116), that the posture of the user (104) is not the target posture based on the walking gait of the user (104) being consistent with the camptocormia posture.

3. The method of any of claims 1-2, wherein:
the signals are first signals,
the method further comprises obtaining, by the processing system (116), one or more second signals that are generated by a wearable device (107) separate from the one or more hearing instruments (102), and
determining whether the posture of the user (104) is the target posture comprises determining, by the processing system (116), whether the posture of the user (104) is the target posture based on the first signals and the second signals;
preferably wherein the wearable device (107) is wearable on a back of the user (104);
preferably wherein the wearable device (107) is a pendant that is wearable around a neck of the user (104).

4. The method of claim 3, wherein the target posture is a neutral spine posture.

5. The method of claim 3, wherein:
determining whether the posture of the user (104) is the target posture comprises determining, by the processing system (116), based on a change to a characteristic of a wireless signal in the second signals, a distance of one or more of the hearing instruments (102) relative to the wearable device (107); and
determining whether the posture of the user (104) is the target posture comprises determining, by the processing system (116), based at least in part on the distance whether the posture of the user (104) is the target posture.

6. The method of any of claims 1-5, wherein generating the information comprises causing the one or more of the hearing instruments (102) to output auditory information regarding the posture of the user (104) to the user (104) while the user (104) is using the hearing instruments (102); and/or
wherein generating the information comprises generating feedback to the user (104) about whether the posture of the user (104) is the target posture; and/or
wherein the hearing instruments (102) are hearing aids.

7. A system comprising:
one or more hearing instruments (102), wherein the one or more hearing instruments (102) include sensors (114);
a processing system (116) comprising one or more processors (112) implemented in circuitry, wherein the one or more processors are configured to:
obtain signals that are generated by or generated based on data from the sensors (114);
the system further comprises one or more storage devices (316) configured to store net displacement values that includes a net displacement value for each degree of freedom in a plurality of degrees of freedom, wherein the net displacement values are derived based on signals generated by inertial measurement units, IMUs, comprised within the one or more sensors of the hearing instruments,
the processing system (116) is configured to:
perform a calibration process, wherein performing the calibration process comprises, based on receiving an indication that the user (104) of the one or more hearing instruments has assumed a target posture, reset the net displacement values;
update the net displacement values based on the signals; and
wherein the one or more processors (112) are further configured to:
determine that the posture of the user (104) is the target posture based on each of the net displacement values being within a respective predefined range;
determine whether the user (104) is asleep and whether the user (104) is in a target posture for sleep, wherein accelerometers comprised within the one or more sensors of the hearing instruments (102) differentiate supine, prone, and upright postures based on directions of gravitational bias; and
generate information based on the user (104) being asleep and not having a target posture for sleeping.

8. The system of claim 7, wherein the hearing instruments (102) include one or more of the processors (112); and/or
wherein the system comprises a computing device (300) that includes one or more of the processors (112), preferably wherein the computing device (300) is one of: a mobile device, a hearing instrument accessory device, a server, or a computer.

9. The system of any of claims 7-8, wherein:
the one or more processors are configured to:
determine, based on the signals from the one or more accelerometers, a current direction of gravity;
based on receiving the indication that the user (104) has assumed the target posture, establish the gravitational bias value for the target posture based on the current direction of gravity; and
after establishing the gravitational bias value for the target posture, update a current gravitational bias value based on subsequent information in the signals, and
determining whether the posture of the user (104) is the target posture comprises determining, by the processing system (116), based on the current gravitational bias value and the gravitational bias value for the target posture, whether the posture of the user (104) is the target posture; and/or
wherein: the sensors (114) include one or more microphones (110), one or more of the one or more hearing instruments (102) includes a speaker (108), the one or more processors are further configured to: cause the speaker (108) to periodically emit a sound, wherein the signals include one or more audio signals detected by the one or more microphones (110); obtaining information, via the one or more microphones (110), indicating reflections of the sound emitted by the speaker (108) in the one or more audio signals; and determine whether the posture of the user (104) is the target posture based in part on a delay of the reflections of the sound; and/or
wherein:
the sensors (114) include a barometer (237),
the signals include a signal from the barometer (237), and the one or more processors are further configured to:
based on receiving the indication that the user (104) has assumed the target posture, determine a target altitude value based on the signal from the barometer (237);
determine, based on the signal from the barometer (237), an altitude of the one or more hearing instruments (102); and
determine whether the posture of the user (104) is the target posture based in part on the altitude of the one or more hearing instruments (102); and/or
wherein the one or more processors are further configured such that, as part of determining whether the posture of the user (104) is the target posture, the one or more processors:
determine, based on one or more of the signals, whether a walking gait of the user (104) is consistent with a camptocormia posture; and
determine that the posture of the user (104) is not the target posture based on the walking gait of the user (104) being consistent with the camptocormia posture.

10. The system of any of claims 7-9, wherein:
the system further comprises a wearable device (107) separate from the one or more hearing instruments (102),
the signals are first signals, the one or more processors are further configured to:
obtain second signals that are generated by the wearable device (107), and determine whether the posture of the user (104) is the target posture based on the first signals and the second signals,
preferably wherein the wearable device (107) is wearable on a back of the user (104).
preferably wherein the wearable device (107) is a pendant that is wearable around a neck of the user (104).

11. The system of claim 10, wherein the target posture is a neutral spine posture.

12. The system of claim 10, wherein:
the one or more processors (112) are configured such that, as part of determining whether the posture of the user (104) is the target posture, the one or more processors (112) determine, based on a change to a characteristic of a wireless signal in the second signals, a distance of one or more of the hearing instruments (102) relative to the wearable device (107); and
determine whether the posture of the user (104) is the target posture comprises determining, by the processing system (116), based at least in part on the distance whether the posture of the user (104) is the target posture.

13. The system of any of claims 7-12, wherein the one or more processors are configured to cause the one or more of the hearing instruments (102) to output auditory information regarding the posture of the user (104) to the user (104) while the user (104) is using the hearing instruments (102); and/or
wherein the one or more processors (112) are configured such that, as part of generating the information about the posture of the user (104), the one or more processors generate feedback to the user (104) about whether the posture of the user (104) is the target posture; and/or
wherein the hearing instruments (102) are hearing aids.

14. A computer-readable medium comprising instructions stored thereon that, when executed, cause one or more processors to:
obtain signals that are generated by or generated based on data from one or more sensors (114) that are included in one or more hearing instruments (102);
store net displacement values that includes a net displacement value for each degree of freedom in a plurality of degrees of freedom, wherein the net displacement values are derived based on signals generated by inertial measurement units, IMUs,
comprised within the one or more sensors of the hearing instruments;
perform a calibration process, wherein performing the calibration process comprises,
based on receiving an indication that the user (104) of the one or more hearing instruments has assumed the target posture, reset the net displacement values;
update the net displacement values based on the signals;
wherein the one or more processors (112) are further configured to:
determine that the posture of the user (104) is the target posture based on each of the net displacement values being within a respective predefined range;
determine whether the user (104) is asleep and whether the user (104) is in a target posture for sleep, wherein accelerometers comprised within the one or more sensors of the hearing instruments (102) differentiate supine, prone, and upright postures based on directions of gravitational bias; and
generate information based on the user (104) being asleep and not having a target posture for sleeping.

15. The computer readable medium of claim 14, wherein execution of the instructions causes the one or more processors to perform the methods of any of claims 2-6.

## Patentansprüche

1. Verfahren, umfassend:
Erhalten von Signalen, die von einem oder mehreren Sensoren (114), die in einem oder mehreren Hörgeräten (102) beinhaltet sind, erzeugt werden oder auf Basis von Daten davon erzeugt werden, durch ein Verarbeitungssystem (116);
Speichern von Netto-Verschiebungswerten, die einen Netto-Verschiebungswert für jeden Freiheitsgrad aus einer Vielzahl von Freiheitsgraden beinhalten, durch das Verarbeitungssystem (116), wobei die Netto-Verschiebungswerte auf Basis von Signalen abgeleitet werden, die von Trägheitsmesseinheiten, IMUs, die innerhalb des einen oder der mehreren Sensoren der Hörgeräte umfasst sind, erzeugt werden;
Durchführen eines Kalibrierungsprozesses, wobei Durchführen des Kalibrierungsprozesses Zurücksetzen der Netto-Verschiebungswerte durch das Verarbeitungssystem (116) auf Basis des Empfangs eines Hinweises, dass der Benutzer (104) des einen oder der mehreren Hörgeräte eine Zielhaltung eingenommen hat, umfasst; und
Aktualisieren der Netto-Verschiebungswerte durch das Verarbeitungssystem (116) auf Basis der Signale;
wobei das Verfahren weiter umfasst:
Bestimmen, ob die Haltung des Benutzers (104) die Zielhaltung ist, auf Basis dessen, dass jeder der Netto-Verschiebungswerte innerhalb eines jeweiligen vordefinierten Bereichs liegt;
Bestimmen, durch das Verarbeitungssystem (116), ob der Benutzer (104) schläft und ob sich der Benutzer (104) in einer Zielhaltung zum Schlafen befindet, wobei Beschleunigungsmesser, die innerhalb des einen oder der mehreren Sensoren der Hörgeräte (102) umfasst sind, auf Basis von Richtungen des Schwerkraftbias zwischen Rückenlage, Bauchlage und aufrechter Haltung unterscheiden; und
Erzeugen von Informationen durch das Verarbeitungssystem (116) auf Basis dessen, dass der Benutzer (104) schläft und keine Zielhaltung zum Schlafen aufweist.

2. Verfahren nach Anspruch 1, wobei:
das Verfahren weiter umfasst:
Bestimmen einer aktuellen Schwerkraftrichtung durch das Verarbeitungssystem (116) auf Basis der Signale von dem einen oder den mehreren Beschleunigungsmessern;
auf Basis des Empfangs des Hinweises, dass der Benutzer (104) die Zielhaltung eingenommen hat, Ermitteln des Schwerkraftbias-Wertes für die Zielhaltung durch das Verarbeitungssystem (116) auf Basis der aktuellen Schwerkraftrichtung; und
nach dem Ermitteln des Schwerkraftbias-Wertes für die Zielhaltung, Aktualisieren eines aktuellen Schwerkraftbias-Wertes durch das Verarbeitungssystem (116) auf Basis von anschließenden Informationen in den Signalen, und
Bestimmen, ob die Haltung des Benutzers (104) die Zielhaltung ist, Bestimmen, ob die Haltung des Benutzers (104) die Zielhaltung ist, durch das Verarbeitungssystem (116), auf Basis des aktuellen Schwerkraftbias-Wertes und des Schwerkraftbias-Wertes für die Zielhaltung umfasst; und/oder
wobei:
die Sensoren (114) ein oder mehrere Mikrofone (110) beinhalten,
eines oder mehrere des einen oder der mehreren Hörgeräte (102) einen Lautsprecher (108) beinhalten,
das Verfahren weiter umfasst:
Bringen des Lautsprechers (108) durch das Verarbeitungssystem (116) dazu, periodisch einen Schall abzugeben, wobei die Signale ein oder mehrere Audiosignale beinhalten, die von dem einen oder den mehreren Mikrofonen (110) erfasst werden;
Erhalten von Informationen, die auf Reflexionen des von dem Lautsprecher (108) abgegebenen Schalls in dem einen oder den mehreren Audiosignalen hinweisen, durch das Verarbeitungssystem (116) über die Mikrofone (110); und
Bestimmen, ob die Haltung des Benutzers (104) die Zielhaltung ist, durch das Verarbeitungssystem (116) zum Teil auf Basis einer Verzögerung der Reflexionen des Schalls; und/oder
wobei:
die Sensoren (114) ein Barometer (237) beinhalten,
die Signale ein Signal von dem Barometer (237) beinhalten, und
das Verfahren weiter umfasst:
auf Basis des Empfangs des Hinweises, dass der Benutzer (104) die Zielhaltung eingenommen hat, Bestimmen eines Zielhöhenwertes durch das Verarbeitungssystem (116) auf Basis des Signals von dem Barometer (237);
Bestimmen einer Höhe des einen oder der mehreren Hörgeräte (102) durch das Verarbeitungssystem (116) auf Basis des Signals von dem Barometer (237); und
Bestimmen, ob die Haltung des Benutzers (104) die Zielhaltung ist, durch das Verarbeitungssystem (116) zum Teil auf Basis der Höhe des einen oder der mehreren Hörgeräte (102); und/oder
wobei das Bestimmen, ob die Haltung des Benutzers (104) die Zielhaltung ist, weiter umfasst:
Bestimmen, ob ein Gangbild des Benutzers (104) mit einer Kamptokormie-Haltung übereinstimmt, durch das Verarbeitungssystem (116) auf Basis eines oder mehrerer der Signale; und
Bestimmen, dass die Haltung des Benutzers (104) nicht die Zielhaltung ist, durch das Verarbeitungssystem (116) auf Basis dessen, dass das Gangbild des Benutzers (104) mit der Kamptokormie-Haltung übereinstimmt.

3. Verfahren nach einem der Ansprüche 1-2, wobei:
die Signale erste Signale sind,
das Verfahren weiter Erhalten eines oder mehrerer zweiter Signale umfasst, die von einer von dem einen oder den mehreren Hörgeräten (102) getrennten, am Körper tragbaren Vorrichtung (107) erzeugt werden, durch das Verarbeitungssystem (116), und
Bestimmen, ob die Haltung des Benutzers (104) die Zielhaltung ist, Bestimmen, ob die Haltung des Benutzers (104) die Zielhaltung ist, durch das Verarbeitungssystem (116) auf Basis der ersten Signale und der zweiten Signale umfasst;
vorzugsweise wobei die am Körper tragbare Vorrichtung (107) auf dem Rücken des Benutzers (104) getragen werden kann;
vorzugsweise wobei die am Körper tragbare Vorrichtung (107) ein Anhänger ist, der um den Hals des Benutzers (104) getragen werden kann.

4. Verfahren nach Anspruch 3, wobei die Zielhaltung eine neutrale Wirbelsäulenhaltung ist.

5. Verfahren nach Anspruch 3, wobei:
Bestimmen, ob die Haltung des Benutzers (104) die Zielhaltung ist, Bestimmen eines Abstands eines oder mehrerer der Hörgeräte (102) relativ zu der am Körper tragbaren Vorrichtung (107) durch das Verarbeitungssystem (116) auf Basis einer Änderung einer Eigenschaft eines drahtlosen Signals in den zweiten Signalen umfasst; und
Bestimmen, ob die Haltung des Benutzers (104) die Zielhaltung ist, Bestimmen, ob die Haltung des Benutzers (104) die Zielhaltung ist, durch das Verarbeitungssystem (116) mindestens zum Teil auf Basis des Abstands umfasst.

6. Verfahren nach einem der Ansprüche 1-5, wobei Erzeugen der Informationen Bringen des einen oder der mehreren der Hörgeräte (102) dazu umfasst, akustische Informationen bezüglich der Haltung des Benutzers (104) an den Benutzer (104) auszugeben, während der Benutzer (104) die Hörgeräte (102) verwendet; und/oder
wobei Erzeugen der Informationen Erzeugen einer Rückmeldung an den Benutzer (104) darüber umfasst, ob die Haltung des Benutzers (104) die Zielhaltung ist; und/oder
wobei die Hörgeräte (102) Hörhilfen sind.

7. System, umfassend:
ein oder mehrere Hörgeräte (102), wobei das eine oder die mehreren Hörgeräte (102) Sensoren (114) beinhalten;
ein Verarbeitungssystem (116), das einen oder mehrere Prozessoren (112) umfasst, die in Schaltungen implementiert sind, wobei der eine oder die mehreren Prozessoren so konfiguriert sind, dass sie:
Signale erhalten, die von den Sensoren (114) erzeugt werden oder auf Basis von Daten davon erzeugt werden;
wobei das System weiter eine oder mehrere Speichervorrichtungen (316) umfasst, die so konfiguriert sind, dass sie Netto-Verschiebungswerte speichern, die einen Netto-Verschiebungswert für jeden Freiheitsgrad aus einer Vielzahl von Freiheitsgraden beinhalten, wobei die Netto-Verschiebungswerte auf Basis von Signalen abgeleitet werden, die von Trägheitsmesseinheiten, IMUs, die innerhalb des einen oder der mehreren Sensoren der Hörgeräte umfasst sind, erzeugt werden,
wobei das Verarbeitungssystem (116) so konfiguriert ist, dass es:
einen Kalibrierungsprozess durchführt, wobei Durchführen des Kalibrierungsprozesses Zurücksetzen der Netto-Verschiebungswerte auf Basis des Empfangs eines Hinweises umfasst, dass der Benutzer (104) des einen oder der mehreren Hörgeräte eine Zielhaltung eingenommen hat;
die Netto-Verschiebungswerte auf Basis der Signale aktualisiert; und
wobei der eine oder die mehreren Prozessoren (112) weiter so konfiguriert sind, dass sie:
bestimmen, dass die Haltung des Benutzers (104) die Zielhaltung ist, auf Basis dessen, dass jeder der Netto-Verschiebungswerte innerhalb eines jeweiligen vordefinierten Bereichs liegt;
bestimmen, ob der Benutzer (104) schläft und ob sich der Benutzer (104) in einer Zielhaltung zum Schlafen befindet, wobei Beschleunigungsmesser, die innerhalb des einen oder der mehreren Sensoren der Hörgeräte (102) umfasst sind, auf Basis von Richtungen des Schwerkraftbias zwischen Rückenlage, Bauchlage und aufrechter Haltung unterscheiden; und
Informationen auf Basis dessen erzeugen, dass der Benutzer (104) schläft und keine Zielhaltung zum Schlafen aufweist.

8. System nach Anspruch 7, wobei die Hörgeräte (102) einen oder mehrere der Prozessoren (112) beinhalten; und/oder
wobei das System eine Rechenvorrichtung (300) umfasst, die einen oder mehrere der Prozessoren (112) beinhaltet, vorzugsweise wobei die Rechenvorrichtung (300) eines ist von: einer mobilen Vorrichtung, einer Hörgeräte-Zubehörvorrichtung, einem Server oder einem Computer.

9. System nach einem der Ansprüche 7-8, wobei:
der eine oder die mehreren Prozessoren so konfiguriert sind, dass sie:
auf Basis der Signale von dem einen oder den mehreren Beschleunigungsmessern eine aktuelle Schwerkraftrichtung bestimmen;
auf Basis des Empfangs des Hinweises, dass der Benutzer (104) die Zielhaltung eingenommen hat, den Schwerkraftbias-Wert für die Zielhaltung auf Basis der aktuellen Schwerkraftrichtung ermitteln; und
nach dem Ermitteln des Schwerkraftbias-Wertes für die Zielhaltung einen aktuellen Schwerkraftbias-Wert auf Basis von anschließenden Informationen in den Signalen aktualisieren, und
Bestimmen, ob die Haltung des Benutzers (104) die Zielhaltung ist, Bestimmen, ob die Haltung des Benutzers (104) die Zielhaltung ist, durch das Verarbeitungssystem (116), auf Basis des aktuellen Schwerkraftbias-Wertes und des Schwerkraftbias-Wertes für die Zielhaltung umfasst; und/oder
wobei: die Sensoren (114) ein oder mehrere Mikrofone (110) beinhalten, eines oder mehrere des einen oder der mehreren Hörgeräte (102) einen Lautsprecher (108) beinhalten, der eine oder die mehreren Prozessoren weiter so konfiguriert sind, dass sie: den Lautsprecher (108) dazu bringen, periodisch einen Schall abzugeben, wobei die Signale ein oder mehrere Audiosignale beinhalten, die von dem einen oder den mehreren Mikrofonen (110) erfasst werden; über das eine oder die mehreren Mikrofone (110) Informationen erhalten, die auf Reflexionen des von dem Lautsprecher (108) abgegebenen Schalls in dem einen oder den mehreren Audiosignalen hinweisen; und zum Teil auf Basis einer Verzögerung der Reflexionen des Schalls bestimmen, ob die Haltung des Benutzers (104) die Zielhaltung ist; und/oder
wobei:
die Sensoren (114) ein Barometer (237) beinhalten,
die Signale ein Signal von dem Barometer (237) beinhalten, und der eine oder die mehreren Prozessoren weiter so konfiguriert sind, dass sie:
auf Basis des Empfangs des Hinweises, dass der Benutzer (104) die Zielhaltung eingenommen hat, einen Zielhöhenwert auf Basis des Signals von dem Barometer (237) bestimmen;
auf Basis des Signals von dem Barometer (237) eine Höhe des einen oder der mehreren Hörgeräte (102) bestimmen; und
zum Teil auf Basis der Höhe des einen oder der mehreren Hörgeräte (102) bestimmen, ob die Haltung des Benutzers (104) die Zielhaltung ist; und/oder
wobei der eine oder die mehreren Prozessoren weiter derart konfiguriert sind, dass als Teil des Bestimmens, ob die Haltung des Benutzers (104) die Zielhaltung ist, der eine oder die mehreren Prozessoren:
auf Basis eines oder mehrerer der Signale bestimmen, ob ein Gangbild des Benutzers (104) mit einer Kamptokormie-Haltung übereinstimmt; und
auf Basis dessen, dass das Gangbild des Benutzers (104) mit der Kamptokormie-Haltung übereinstimmt, bestimmen, dass die Haltung des Benutzers (104) nicht die Zielhaltung ist.

10. System nach einem der Ansprüche 7-9, wobei:
das System weiter eine von dem einen oder den mehreren Hörgeräten (102) getrennte, am Körper tragbare Vorrichtung (107) umfasst,
die Signale erste Signale sind, der eine oder die mehreren Prozessoren weiter so konfiguriert sind, dass sie: zweite Signale erhalten, die von der am Körper tragbaren Vorrichtung (107) erzeugt werden, und auf Basis der ersten Signale und der zweiten Signale bestimmen, ob die Haltung des Benutzers (104) die Zielhaltung ist,
vorzugsweise wobei die am Körper tragbare Vorrichtung (107) auf dem Rücken des Benutzers (104) getragen werden kann,
vorzugsweise wobei die am Körper tragbare Vorrichtung (107) ein Anhänger ist, der um den Hals des Benutzers (104) getragen werden kann.

11. System nach Anspruch 10, wobei die Zielhaltung eine neutrale Wirbelsäulenhaltung ist.

12. System nach Anspruch 10, wobei:
der eine oder die mehreren Prozessoren (112) derart konfiguriert sind, dass als Teil des Bestimmens, ob die Haltung des Benutzers (104) die Zielhaltung ist, der eine oder die mehreren Prozessoren (112) auf Basis einer Änderung einer Eigenschaft eines drahtlosen Signals in den zweiten Signalen einen Abstand eines oder mehrerer der Hörgeräte (102) relativ zu der am Körper tragbaren Vorrichtung (107) bestimmen; und
Bestimmen, ob die Haltung des Benutzers (104) die Zielhaltung ist, Bestimmen, ob die Haltung des Benutzers (104) die Zielhaltung ist, durch das Verarbeitungssystem (116) mindestens zum Teil auf Basis des Abstands umfasst.

13. System nach einem der Ansprüche 7-12, wobei der eine oder die mehreren Prozessoren so konfiguriert sind, dass sie das eine oder die mehreren der Hörgeräte (102) dazu bringen, akustische Informationen bezüglich der Haltung des Benutzers (104) an den Benutzer (104) auszugeben, während der Benutzer (104) die Hörgeräte (102) verwendet; und/oder
wobei der eine oder die mehreren Prozessoren (112) derart konfiguriert sind, dass als Teil des Erzeugens der Informationen über die Haltung des Benutzers (104) der eine oder die mehreren Prozessoren eine Rückmeldung an den Benutzer (104) darüber erzeugen, ob die Haltung des Benutzers (104) die Zielhaltung ist; und/oder
wobei die Hörgeräte (102) Hörhilfen sind.

14. Computerlesbares Medium, das darauf gespeicherte Anweisungen umfasst, die, wenn sie ausgeführt werden, einen oder mehrere Prozessoren dazu bringen:
Signale zu erhalten, die von einem oder mehreren Sensoren (114), die in einem oder mehreren Hörgeräten (102) beinhaltet sind, erzeugt werden oder auf Basis von Daten davon erzeugt werden;
Netto-Verschiebungswerte zu speichern, die einen Netto-Verschiebungswert für jeden Freiheitsgrad aus einer Vielzahl von Freiheitsgraden beinhalten, wobei die Netto-Verschiebungswerte auf Basis von Signalen abgeleitet werden, die von Trägheitsmesseinheiten, IMUs, die innerhalb des einen oder der mehreren Sensoren der Hörgeräte umfasst sind, erzeugt werden;
einen Kalibrierungsprozess durchzuführen, wobei Durchführen des Kalibrierungsprozesses Zurücksetzen der Netto-Verschiebungswerte auf Basis des Empfangs eines Hinweises umfasst, dass der Benutzer (104) des einen oder der mehreren Hörgeräte die Zielhaltung eingenommen hat;
die Netto-Verschiebungswerte auf Basis der Signale aktualisieren;
wobei der eine oder die mehreren Prozessoren (112) weiter so konfiguriert sind, dass sie:
bestimmen, dass die Haltung des Benutzers (104) die Zielhaltung ist, auf Basis dessen, dass jeder der Netto-Verschiebungswerte innerhalb eines jeweiligen vordefinierten Bereichs liegt;
bestimmen, ob der Benutzer (104) schläft und ob sich der Benutzer (104) in einer Zielhaltung zum Schlafen befindet, wobei Beschleunigungsmesser, die innerhalb des einen oder der mehreren Sensoren der Hörgeräte (102) umfasst sind, auf Basis von Richtungen des Schwerkraftbias zwischen Rückenlage, Bauchlage und aufrechter Haltung unterscheiden; und
Informationen auf Basis dessen erzeugen, dass der Benutzer (104) schläft und keine Zielhaltung zum Schlafen aufweist.

15. Computerlesbares Medium nach Anspruch 14, wobei Ausführung der Anweisungen den einen oder die mehreren Prozessoren dazu bringt, die Verfahren nach einem der Ansprüche 2-6 durchzuführen.

## Revendications

1. Procédé comprenant :
l'obtention, par un système de traitement (116), de signaux générés par ou générés à partir de données provenant d'un ou plusieurs capteurs (114) inclus dans un ou plusieurs instruments auditifs (102) ;
le stockage, par le système de traitement (116), de valeurs de déplacement net qui incluent une valeur de déplacement net pour chaque degré de liberté dans une pluralité de degrés de liberté, dans lequel les valeurs de déplacement net sont dérivées sur la base de signaux générés par des unités de mesure inertielle, IMU, comprises dans les un ou plusieurs capteurs des instruments auditifs ;
la réalisation d'un processus d'étalonnage, dans lequel la réalisation du processus d'étalonnage comprend, sur la base d'une indication selon laquelle l'utilisateur (104) des un ou plusieurs instruments auditifs a adopté une posture cible, la réinitialisation, par le système de traitement (116), des valeurs de déplacement net ; et
la mise à jour, par le système de traitement (116), des valeurs de déplacement net basées sur les signaux ;
dans lequel le procédé comprend en outre :
la détermination si la posture de l'utilisateur (104) est la posture cible en fonction de chacune des valeurs de déplacement net se trouvant dans une plage prédéfinie respective ;
la détermination, par le système de traitement (116), si l'utilisateur (104) dort et si l'utilisateur (104) se trouve dans une posture cible pour le sommeil, dans lequel les accéléromètres intégrés aux un ou plusieurs capteurs des instruments auditifs (102) différencient les postures couchée sur le dos, couchée sur le ventre et debout en fonction des directions du biais gravitationnel ; et
la génération, par le système de traitement (116), d'informations basées sur le fait que l'utilisateur (104) est endormi et ne présente pas de posture cible pour dormir.

2. Procédé selon la revendication 1, dans lequel :
le procédé comprend en outre :
la détermination, par le système de traitement (116), sur la base des signaux provenant d'un ou plusieurs accéléromètres, d'une direction actuelle de la gravité ;
sur la base de la réception de l'indication selon laquelle l'utilisateur (104) a adopté la posture cible, l'établissement, par le système de traitement (116), de la valeur du biais gravitationnel pour la posture cible en fonction de la direction actuelle de la gravité ; et
après avoir établi la valeur du biais gravitationnel pour la posture cible, la mise à jour, par le système de traitement (116), d'une valeur de biais gravitationnel actuelle basée sur des informations ultérieures contenues dans les signaux, et
la détermination si la posture de l'utilisateur (104) est la posture cible comprend la détermination, par le système de traitement (116), sur la base de la valeur actuelle du biais gravitationnel et de la valeur du biais gravitationnel pour la posture cible, si la posture de l'utilisateur (104) est la posture cible ; et/ou
dans lequel :
les capteurs (114) incluent un ou plusieurs microphones (110),
un ou plusieurs des un ou plusieurs instruments auditifs (102) incluent un haut-parleur (108),
le procédé comprend en outre :
l'incitation, par le système de traitement (116), du haut-parleur (108) à émettre périodiquement un son, dans lequel les signaux incluent un ou plusieurs signaux audio détectés par les un ou plusieurs microphones (110) ;
l'obtention d'informations, par le système de traitement (116) via les microphones (110), indiquant les réflexions du son émis par le haut-parleur (108) dans les un ou plusieurs signaux audio ; et
la détermination, par le système de traitement (116), si la posture de l'utilisateur (104) est la posture cible en se basant en partie sur un retard des réflexions du son ; et/ou
dans lequel :
les capteurs (114) incluent un baromètre (237),
les signaux incluent un signal provenant du baromètre (237), et
le procédé comprend en outre :
sur la base de la réception de l'indication que l'utilisateur (104) a adopté la posture cible, la détermination, par le système de traitement (116), d'une valeur d'altitude cible basée sur le signal du baromètre (237) ;
la détermination, par le système de traitement (116), à partir du signal du baromètre (237), de l'altitude des un ou plusieurs instruments auditifs (102) ; et
la détermination, par le système de traitement (116), si la posture de l'utilisateur (104) est la posture cible en fonction en partie de l'altitude des un ou plusieurs instruments auditifs (102) ; et/ou
dans lequel la détermination du caractère cible de la posture de l'utilisateur (104) comprend en outre :
la détermination, par le système de traitement (116), sur la base d'un ou plusieurs signaux, si la démarche de l'utilisateur (104) est compatible avec une posture de camptocormie ; et
la détermination, par le système de traitement (116), que la posture de l'utilisateur (104) n'est pas la posture cible en se basant sur la démarche de l'utilisateur (104) qui est cohérente avec la posture de camptocormie.

3. Procédé selon l'une quelconque des revendications 1-2, dans lequel :
les signaux sont des premiers signaux,
le procédé comprend en outre l'obtention, par le système de traitement (116), d'un ou plusieurs deuxièmes signaux générés par un dispositif portable (107) distincts de ceux provenant des un ou plusieurs instruments auditifs (102), et
la détermination si la posture de l'utilisateur (104) est la posture cible comprend la détermination, par le système de traitement (116), si la posture de l'utilisateur (104) est la posture cible sur la base des premiers signaux et des deuxièmes signaux ;
de préférence dans lequel le dispositif portable (107) est porté sur le dos de l'utilisateur (104) ;
de préférence dans lequel le dispositif portable (107) est un pendentif qui peut être porté autour du cou de l'utilisateur (104).

4. Procédé selon la revendication 3, dans lequel la posture cible est une posture de colonne vertébrale neutre.

5. Procédé selon la revendication 3, dans lequel :
la détermination si la posture de l'utilisateur (104) correspond à la posture cible comprend la détermination, par le système de traitement (116), sur la base d'une modification d'une caractéristique d'un signal sans fil dans les deuxièmes signaux, d'une distance d'un ou plusieurs des instruments auditifs (102) par rapport au dispositif portable (107) ; et
la détermination si la posture de l'utilisateur (104) correspond à la posture cible comprend la détermination, par le système de traitement (116), sur la base au moins en partie de la distance, si la posture de l'utilisateur (104) est la posture cible.

6. Procédé selon l'une quelconque des revendications 1-5, dans lequel la génération des informations comprend le fait de faire en sorte que les un ou plusieurs des instruments auditifs (102) produisent des informations auditives concernant la posture de l'utilisateur (104) audit utilisateur (104) pendant que l'utilisateur (104) utilise les instruments auditifs (102) ; et/ou
dans lequel la génération des informations comprend la génération de retours d'information à l'utilisateur (104) sur la question de savoir si la posture de l'utilisateur (104) est la posture cible ; et/ou
dans lequel les instruments auditifs (102) sont des aides auditives.

7. Système comprenant :
un ou plusieurs instruments auditifs (102), dans lequel les un ou plusieurs instruments auditifs (102) incluent des capteurs (114) ;
un système de traitement (116) comprenant un ou plusieurs processeurs (112) mis en œuvre dans des circuits, dans lequel les un ou plusieurs processeurs sont configurés pour :
obtenir des signaux générés par ou générés à partir de données provenant des capteurs (114) ;
le système comprend en outre un ou plusieurs dispositifs de stockage (316) configurés pour stocker des valeurs de déplacement net qui incluent une valeur de déplacement net pour chaque degré de liberté dans une pluralité de degrés de liberté, dans lequel les valeurs de déplacement net sont dérivées de signaux générés par des unités de mesure inertielle, IMU, comprises dans les un ou plusieurs capteurs des instruments auditifs,
le système de traitement (116) est configuré pour :
effectuer un processus d'étalonnage, dans lequel l'exécution du processus d'étalonnage comprend, sur la base de la réception d'une indication que l'utilisateur (104) des un ou plusieurs instruments auditifs a adopté une posture cible, la réinitialisation des valeurs de déplacement net ;
mettre à jour les valeurs de déplacement net sur la base des signaux ; et
dans lequel le ou les processeurs (112) sont en outre configurés pour :
déterminer que la posture de l'utilisateur (104) est la posture cible sur la base de chacune des valeurs de déplacement net se trouvant dans une plage prédéfinie respective ;
déterminer si l'utilisateur (104) dort et si l'utilisateur (104) se trouve dans une posture cible pour le sommeil, dans lequel les accéléromètres intégrés au ou aux capteurs des instruments auditifs (102) différencient les postures couchée sur le dos, couchée sur le ventre et debout en fonction des directions du biais gravitationnel ; et
générer des informations basées sur le fait que l'utilisateur (104) est endormi et ne présente pas de posture cible pour dormir.

8. Système selon la revendication 7, dans lequel les instruments auditifs (102) incluent un ou plusieurs des processeurs (112) ; et/ou
dans lequel le système comprend un dispositif informatique (300) qui inclut un ou plusieurs processeurs (112), de préférence dans lequel le dispositif informatique (300) est l'un des suivants : un dispositif mobile, un accessoire pour instrument auditif, un serveur ou un ordinateur.

9. Système selon l'une quelconque des revendications 7-8, dans lequel :
les un ou plusieurs processeurs sont configurés pour :
déterminer, à partir des signaux provenant des un ou plusieurs accéléromètres, une direction actuelle de la gravité ;
en se basant sur l'indication que l'utilisateur (104) a adopté la posture cible, établir la valeur du biais gravitationnel pour la posture cible en fonction de la direction actuelle de la gravité ; et
après avoir établi la valeur du biais gravitationnel pour la posture cible, mettre à jour une valeur de biais gravitationnel actuelle en fonction des informations ultérieures contenues dans les signaux, et
la détermination si la posture de l'utilisateur (104) est la posture cible comprend la détermination, par le système de traitement (116), sur la base de la valeur actuelle du biais gravitationnel et de la valeur du biais gravitationnel pour la posture cible, si la posture de l'utilisateur (104) est la posture cible ; et/ou
dans lequel : les capteurs (114) incluent un ou plusieurs microphones (110), un ou plusieurs des un ou plusieurs instruments auditifs (102) incluent un haut-parleur (108), les un ou plusieurs processeurs sont en outre configurés pour : amener le haut-parleur (108) à émettre périodiquement un son, dans lequel les signaux incluent un ou plusieurs signaux audio détectés par les un ou plusieurs microphones (110) ; obtenir des informations, via les un ou plusieurs microphones (110), indiquant des réflexions du son émis par le haut-parleur (108) dans les un ou plusieurs signaux audio ; et déterminer si la posture de l'utilisateur (104) est la posture cible en se basant notamment sur le délai des réflexions du son ; et/ou
dans lequel :
les capteurs (114) incluent un baromètre (237),
les signaux incluent un signal provenant du baromètre (237), et les un ou plusieurs processeurs sont en outre configurés pour :
sur la base de l'indication que l'utilisateur (104) a adopté la posture cible, déterminer une valeur d'altitude cible sur la base du signal du baromètre (237) ;
déterminer, à partir du signal du baromètre (237), l'altitude des un ou plusieurs instruments auditifs (102) ; et
déterminer si la posture de l'utilisateur (104) est la posture cible en se basant en partie sur l'altitude des un ou plusieurs instruments auditifs (102) ; et/ou
dans lequel les un ou plusieurs processeurs sont en outre configurés de telle sorte que, dans le cadre de la détermination si la posture de l'utilisateur (104) est la posture cible, les un ou plusieurs processeurs :
déterminent, sur la base d'un ou plusieurs des signaux, si la démarche de l'utilisateur (104) est compatible avec une posture de camptocormie ; et
déterminent que la posture de l'utilisateur (104) n'est pas la posture cible en se basant sur la démarche de l'utilisateur (104) qui est cohérente avec la posture de camptocormie.

10. Système selon l'une quelconque des revendications 7-9, dans lequel :
le système comprend en outre un dispositif portable (107) distinct des un ou plusieurs instruments auditifs (102),
les signaux sont des premiers signaux, les un ou plusieurs processeurs sont en outre configurés pour : obtenir des deuxièmes signaux générés par le dispositif portable (107), et déterminer si la posture de l'utilisateur (104) est la posture cible en fonction des premiers signaux et des deuxièmes signaux,
de préférence dans lequel le dispositif portable (107) est porté sur le dos de l'utilisateur (104).
de préférence dans lequel le dispositif portable (107) est un pendentif qui peut être porté autour du cou de l'utilisateur (104).

11. Système selon la revendication 10, dans lequel la posture cible est une posture de colonne vertébrale neutre.

12. Système selon la revendication 10, dans lequel :
les un ou plusieurs processeurs (112) sont configurés de telle sorte que, dans le cadre de la détermination si la posture de l'utilisateur (104) est la posture cible, les un ou plusieurs processeurs (112) déterminent, sur la base d'une modification d'une caractéristique d'un signal sans fil dans les deuxièmes signaux, une distance des un ou plusieurs des instruments auditifs (102) par rapport au dispositif portable (107) ; et
la détermination si la posture de l'utilisateur (104) est la posture cible comprend la détermination, par le système de traitement (116), au moins en partie sur la distance, si la posture de l'utilisateur (104) est la posture cible.

13. Système selon l'une quelconque des revendications 7-12, dans lequel les un ou plusieurs processeurs sont configurés pour faire en sorte que les un ou plusieurs instruments auditifs (102) produisent des informations auditives concernant la posture de l'utilisateur (104) à l'utilisateur (104) pendant que celui-ci utilise les instruments auditifs (102) ; et/ou
dans lequel les un ou plusieurs processeurs (112) sont configurés de telle sorte que, dans le cadre de la génération des informations sur la posture de l'utilisateur (104), les un ou plusieurs processeurs génèrent un retour d'information à l'utilisateur (104) sur la question de savoir si la posture de l'utilisateur (104) est la posture cible ; et/ou
dans lequel les instruments auditifs (102) sont des aides auditives.

14. Support lisible par ordinateur comprenant des instructions qui, lorsqu'elles sont exécutées, amènent un ou plusieurs processeurs à :
obtenir des signaux générés par ou générés à partir de données provenant d'un ou plusieurs capteurs (114) inclus dans un ou plusieurs instruments auditifs (102) ;
stocker des valeurs de déplacement net qui incluent une valeur de déplacement net pour chaque degré de liberté dans une pluralité de degrés de liberté, dans lequel les valeurs de déplacement net sont dérivées de signaux générés par des unités de mesure inertielle, IMU, comprises dans les un ou plusieurs capteurs des instruments auditifs ;
effectuer un processus d'étalonnage, dans lequel l'exécution du processus d'étalonnage comprend, sur la base de la réception d'une indication que l'utilisateur (104) des un ou plusieurs instruments auditifs a adopté la posture cible, la réinitialisation des valeurs de déplacement net ;
mettre à jour les valeurs de déplacement net sur la base des signaux ;
dans lequel les un ou plusieurs processeurs (112) sont en outre configurés pour :
déterminer que la posture de l'utilisateur (104) est la posture cible sur la base de chacune des valeurs de déplacement net se trouvant dans une plage prédéfinie respective ;
déterminer si l'utilisateur (104) dort et si l'utilisateur (104) se trouve dans une posture cible pour le sommeil, dans lequel les accéléromètres intégrés au ou aux capteurs des instruments auditifs (102) différencient les postures couchée sur le dos, couchée sur le ventre et debout en fonction des directions du biais gravitationnel ; et
générer des informations basées sur le fait que l'utilisateur (104) est endormi et ne présente pas de posture cible pour dormir.

15. Support lisible par ordinateur selon la revendication 14, dans lequel l'exécution des instructions amène le ou les processeurs à exécuter les procédés selon l'une quelconque des revendications 2-6.
